# EUROPEAN PATENT SPECIFICATION

(11) **EP 3 191 188 B1**
(45) Date of publication and mention of the grant of the patent: **03.11.2021**
(21) Application number: 14901662.8
(22) Date of filing: 12.09.2014
(51) Int. Cl.: A61Q 1/10, A61K 8/42, A61K 8/44, A61K 8/89, A61K 8/92, A61K 8/06, A61K 8/19

(54) **COMPOSITIONS FOR KERATIN FIBERS**
ZUSAMMENSETZUNGEN FÜR KERATINFASERN
COMPOSITION POUR FIBRES DE KÉRATINE

(43) Date of publication of application: 19.07.2017
(73) Proprietor: Avon Products, Inc., Suffern, NY 10901 (US)
(72) Inventor: ABDO, Mohannad, Clifton, New Jersey 07013 (US); NOVACK, Candice DeLeo, Monroe, New York 10950 (US); HOWELL, Ashley L., Oakland, New Jersey 07436 (US); EBANKS, Jody P., Nyack, New York 10960 (US)
(74) Representative: Abel & Imray
(86) International application number: PCT/US2014/055407
(87) International publication number: WO 2016/039771

(56) References cited:
- EP-A1- 0 832 637
- EP-A1- 1 870 075
- US-A1- 2010 086 507
- US-A1- 2012 298 128
- US-B2- 7 632 489
- US-B2- 7 820 151
- US-B2- 8 136 536
- US-B2- 8 263 055
- US-B2- 8 591 871
- DATABASE GNPD [Online] MINTEL; 1 November 2000 (2000-11-01), "Mascara", XP002777390, Database accession no. 77923
- DATABASE GNPD [Online] MINTEL; 3 November 2000 (2000-11-03), anonymous: "Mascara", XP055776926, retrieved from https://www.gnpd.com/sinatra/recordpage/77 923/ Database accession no. 77923

## Description

### FIELD OF INVENTION

The present invention relates generally to topical compositions and methods of using the compositions to treat keratin fibers. The compositions, such as mascaras, can form films of low resiliency on the surface of the keratin fibers, which impart moldability to the treated fibers, thereby enhancing the range of styling effects and options available to the user.

### BACKGROUND OF THE INVENTION

Mascaras are generally used by women to accentuate their lashes - that is, impart color and/or aesthetic effects to eyelashes. In particular, consumers look to mascaras to darken, lengthen, thicken, and curl their lashes. Most mascara compositions contain waxes as a means to obtain all of these attributes.

However, waxes have certain undesirable properties when used in a mascara composition. Most notably, waxes are typically opaque solids. Mascaras containing such waxes exhibit more muted colors or require additional amounts of pigments to achieve the same color effect, thus, impairing the mascara's ability to provide the dark coloring desired by consumers. Accordingly, mascara compositions that impart a richer, more lustrous appearance than conventional mascaras and that provide depth of color (deep dark coloring), while maintaining or improving any of the other desired attributes of mascara are needed. It would further be desirable to have mascara that has the visual appearance of lengthening and thickening eyelashes while imparting a dramatic color effect. Moreover, most conventional mascaras, once they are set and form a cured film, behave only elastically. For example, the treated lash can be deformed, but when the force is removed, it snaps back to the original position. It would be desirable to develop a mascara that behaves viscoelastically, such that it is deformable and moldable. This would allow a user to apply the mascara, and then after the mascara has dried to form a cured film, it could be molded into particular styles.

### SUMMARY OF THE INVENTION

The present invention provides a method for styling keratin fibers (e.g., eyelashes) as defined in claim 1 and a cosmetic composition as defined in claim 12. The method comprises applying a composition (e.g., a pigmented cosmetic such as a mascara) to form a film on at least a portion of surfaces of the fibers, allowing the film to dry (i.e., partial or complete evaporation of volatile solvents) to form a moldable film thereon, and applying a force to the treated fibers to mold the treated fiber into a desired configuration. As used herein, the term "moldable" refers to a dry film with less than 100% resilience (more typically, less than 75%, or less than 50%, or less than 25%, or less than 20%, or less than 15%, or less than 12%, or less than 10%, or less than 8% resilience) when displaced 2.0 mm by a compressive force at 25°C. The film of the method of the present invention is characterized, upon evaporation of solvents, by a resilience of less than 15% when displaced 2.0 mm by a compression force at 25°C. The user may then mold the treated keratin fibers into a desired configuration (e.g., curled, straight, etc.), by applying a force to the treated fibers (e.g., by pressing, brushing, crimping, bending, etc.). After the force is removed, the keratin fibers remain substantially in the desired configuration due to the fact that the film behaves non-elastically. The treated keratin fibers may be subsequently re-molded into a second desired configuration by applying a second force to the fibers. After the force is removed, the keratin fibers remain in the second desired configuration. The compositions (e.g., mascara compositions) comprise a wax component (e.g., one or more low opacity waxes), an oil phase gellant, one or more pigments and/or fillers, and an oil capable of forming a gel with the gellants. The low opacity wax component may comprise one or more waxes, and will be characterized by a low opacity, such as a ΔL* value for each wax or in the aggregate for all waxes, of less than 8 (e.g., less than 7, less than 6, or less than 5). Each wax, individually, may also have a ΔL* value of less than 8 (e.g., less than 7, less than 6, or less than 5). The wax component comprises between 7.5% and 30% by weight of the weight of the entire composition (e.g., 7.5% to 15%, or 10% to 12.5% by weight). The wax component may comprise silicone wax, or it may be predominantly comprised of silicone wax, or it may consist essentially of silicone wax, or it may consist of silicone wax. A wax component that "consists essentially of" silicone wax excludes additional waxes in amount that have a significant (e.g., > 10%) effect on either the resiliency or the transparency of the film. The wax component may consist predominantly of, or consist essentially of, a combination of silicone wax and beeswax. In other embodiments, the wax component may comprise one or more of carnauba wax, beeswax, Ozokerite, and silicone wax. The gellant is an oil phase gellant (e.g., comprising one or more amide-based gellants, and in particular glutamide-based gellants, such as dibutyl laurolyl glutamide and/or dibutyl ethylhexanoyl glutamide) in an amount between about 0. 1% and about 1% (e.g., between 0.1% and 0.5%). The fillers and/or pigments may be present in an amount greater than 0.01%, or greater than 0.1%, or greater than 1%, or greater than 2.5%, or greater than 5%, and less than 15%, less than 10%, less than 7%, less than 6%, less than 5%, less about 4%, less than 3%, less than 2%, less than 1%, or less than 0.5% by weight). Typically, the compositions will comprise one or both of carbon black and iron oxides, each of which may be present, individually or in the aggregate with all other particulates, in the foregoing amounts. In some embodiments, the pigment is not a white pigment (e.g., it is not zinc oxide, calcium carbonate, or titanium dioxide). The oil is capable of forming a gel with the oil phase gellant, and is present in an amount between 1% and 10%, e.g. between 3% and 10%, or between 5% and 8%. The compositions may be free of polyamide gellants and/or polyester amide gellants. The compositions may comprise an aqueous phase, and may be in the form of emulsions, or they may be substantially anhydrous (i.e., < 2% water), or they may be anhydrous.

These and other aspects of the present invention will become apparent to those skilled in the art according to the present description, including the figures and appended claims.

### BRIEF DESCRIPTION OF THE FIGURES

FIG. 1 shows the color travel of three mascara compositions comprising low opacity waxes compared to a commercial mascara comprising high opacity (ΔL* value > 8) waxes.
FIG. 2 shows the multi-angle chroma of three mascara compositions compared to a commercial mascara comprising high opacity (ΔL* value > 8) waxes.
FIG. 3 shows the payoff (weight deposition in grams) of three mascara compositions compared to a commercial mascara comprising high opacity (ΔL* value > 8) waxes.
FIG. 4 shows the payoff (weight deposition in grams), of six different mascara compositions, each one comprised of a different wax.

### DETAILED DESCRIPTION

As used herein, the term "consisting essentially of" is intended to limit the invention to the specified materials or steps and those materials or steps that do not materially affect the basic and novel characteristics of the claimed invention, for example, resiliency if a dried film and/or loss of transparency and translucency, as understood from a reading of this specification. When used in relation to the low opacity waxes, the phrase "consisting essentially of" excludes the presence of high opacity waxes in amounts that give a measurable reduction in chroma or color travel, and/or significantly affect the resiliency of a dried film.

The terms "a" and "an", as used herein and in the appended claims, mean "one or more" unless otherwise indicated herein.

It should be noted that unless indicated to the contrary, as used herein, percent (%) is % by weight, based on the total weight of the composition (including any solvents or vehicle).

The compositions of the invention are intended for application to keratin fibers (e.g., lashes, hair of the scalp, etc.). In some embodiments, the compositions are color cosmetics such as mascaras that comprise pigments and may further comprise lakes, and/or dyes. The compositions may comprise volatile solvents to promote application and spreadability. The solvents or other vehicle is topically acceptable, by which is meant non-toxic and substantially non-irritating to human integuments.

The compositions are applied to keratin fibers, for example with a brush, comb, or other suitable applicator (including any known mascara applicator). The composition is applied to form a film or coating on the surface of individual fibers (e.g., lashes) along part or all of the length of the fiber. After any volatile solvents have evaporated, a dried film is produced on the treated fibers. The dried film may advantageously be characterized by unique physical properties heretofore unknown in conventional lash treatments. In particular, the dried films are not entirely elastic, such that on deformation by a force, the original shape is not fully (or even substantially) recovered after the force is removed. In other words, the dried films, and consequently the treated fibers, are moldable.

The topical compositions may comprise low opacity waxes that may exhibit excellent color attributes, high chroma, high color travel, and enhanced depth of color. The compositions may simultaneously comprise a high level of waxes (e.g., greater than 10% by weight) while maintaining a suitable viscosity for liquid cosmetics such as mascaras. The compositions ideally deliver an unexpectedly high payoff (weight deposition) when applied to lashes. When the compositions are applied in the form of a mascara to the eyelashes, the volumizing and lengthening effects of the mascara is advantageously not impaired.

The compositions of the invention typically comprise a wax component and one or more pigments. The cosmetic compositions further comprise a gel component. The compositions may have a viscosity between about 250,000 and about 2,000,000 mPa.s (cps), (measured at a spindle rate of 4 rpm and 25°C) and have a payoff value greater than an otherwise identical composition that lacks either the gel component or the wax component.

The composition may be, for example, in the form of an emulsion, or it may be an anhydrous composition. In one aspect, the composition is a liquid, for example, one having a viscosity from about 250,000 to about 2,000,000. The one or more pigments may comprise any suitable pigments (e.g., iron oxide black and/or carbon black), or lakes. In some embodiments, the pigment is not a white pigment (e.g., it is not zinc oxide, calcium carbonate, or titanium dioxide).

The wax component may comprise one or more low opacity waxes. Waxes useful in the compositions of the present invention may include natural, mineral, or synthetic waxes exhibiting low opacity (e.g., a ΔL* less than 8 as determined by the procedure set forth in Example 1 below). The waxes collectively may have a ΔL* value of less than 8 and/or each individual wax may have a ΔL* value of less than 8. In other embodiments the ΔL* value of the waxes, individually is 10 or less, 9 or less, 8 or less, 6 or less, 5 or less, 4 or less, 3 or less, 2 or less, or 1 or less. In certain embodiments the wax component may comprise one or more individual waxes having a ΔL* less than 8, in combination with one or more waxes individually having a ΔL* of 8 or greater, as long as in the aggregate, the combination of waxes (i.e., the wax component) exhibits a ΔL* less than 8. In one embodiment, the wax component does not comprise an individual wax having a ΔL* 8 or greater. In other embodiments the wax component does not comprise more than 15%, more than 10%, or more than 5% of a wax having a ΔL* value of 8 or greater, by weight of the wax component. In one embodiment, the wax component has a ΔL* value of less than 8.

ΔL* is measured by measuring L* values on a drawdown film of mascara on a black Leneta card using a hand-held spectrophotometer (e.g., a Konica Minolta CM-2600d spectrophotometer). The drawdown film is obtained by applying 3 mL of the sample (i.e., in the case of an emulsion, the non-aqueous phase or the gel base into which wax(es) or filler(s) have been incorporated, as the case may be, otherwise the cosmetic base or the cosmetic base into which wax(es) or filler(s) have been incorporated, as the case may be) to obtain a test film on the Leneta card that is about 75 microns in thickness and allowed to dry for 2 hours. The Leneta card itself is the standard for the color black in the tristimulus color measurement method, and by definition has an L value of zero.

Suitable low opacity waxes include, but are not limited to, carnauba wax, beeswax, bleached beeswax, ozokerite, kahlwax 7307, Silwax CRM2, Silwax 5022, Silwax L118. Silwax D221M, Silwax Di-5026, POE (20M) sorbitol beeswax, PEG-8 beeswax, low opacity variants and combinations thereof.

The wax component comprises between 7.5% and 30% by weight, or 7.5% to 15%, or 10% to 12%, or about 11% by weight of the composition.

The wax component may comprise 10% by weight or more of the weight of the entire composition (e.g., 10% to 30%, or 11% to 25%, or 12% to 20%, or more, by weight).

The wax component may comprise, for example, silicone wax, or it may consist predominantly of silicone wax, or it may consist essentially of silicone wax, or it may consist of silicone wax. In another aspect, the wax component may consist predominantly of, or consist essentially of, silicone wax and beeswax. The wax component comprises one or more of carnauba wax, beeswax, Ozokerite, and silicone wax.

In one embodiment, the wax component comprises silicone wax (e.g., from 1% to 12% by weight) and beeswax (e.g., from 1% to 12% by weight). In another embodiment, the wax component consists predominantly of silicone wax and beeswax. In another embodiment, the wax component consists essentially of silicone wax and beeswax. In another embodiment, the wax component consists of silicone wax and beeswax. In one embodiment, the composition comprises less than 2% by weight or less than 1% by weight high opacity waxes. In one embodiment, the composition is free of high opacity waxes.

In another embodiment, the wax component comprises one or more silicone waxes. In another embodiment, the wax component consists predominantly of silicone wax. In another embodiment, the wax component consists essentially of silicone wax. In another embodiment, the wax component consists of silicone wax. In a further embodiment, the silicone wax or silicone waxes comprise the majority of wax component of the composition.

In one preferred embodiment, cosmetic compositions are provided that comprise at least 10% by weight of a combination of silicone wax and beeswax, and one or more pigments. In some embodiments, the wax component consists predominantly of silicone wax and beeswax, and in another embodiment consists essentially of silicone wax and beeswax. In one preferred embodiment, the composition is a liquid and has a viscosity between about 250,000 mPa.s (cps) and about 2,000,000 mPa.s (cps).

The wax component is present in the cosmetic compositions in an amount between 7.5% to 30%, e.g. 10% to 25%, or 15% to 20% by weight of the composition. In some embodiments, the wax component may comprise 10% or more, 11% or more, 12 % or more, 13% or more, 14% or more, 15% or more, or 20% or more, by weight of the composition. The wax component may also comprise about 10%, about 11%, about 12%, about 13%, about 14%, about 15%, about 16%, about 17%, about 18%, about 19%, about 20%, about 21%, about 22%, about 23%, about 24%, or about 24.5% of the cosmetic composition.

In the case of mascara compositions, certain wax components may impart the desired volume and lengthening to the mascara without adversely impacting, for example, the color depth of the composition. These waxes may be individually present in the compositions in the following weight percentages: carnauba waxes may be present in an amount from 1 to 30%, 10 to 30%, or 15 to 25%; beeswax may be present an amount from 1 to 30%, 2.5 to 20%, 5 to 15%, or 7.5 to 12.5%; Ozokerite wax may be present an amount from 0.1 to 15%, 1 to 10%, or 1.5 to 5%; silicone waxes, such as Silwax 5022, Silwax D221M, and Silwax L118 may be present an amount from 0.1 to 20%, 1 to 10%, or 1.5 to 5%. In a further embodiment, the wax component may be comprised of carnauba wax an amount from about 1 to about 20%, Kahlwax 7307 in an amount from 1 to 10%, beeswax an amount from 1 10%; Ozokerite an amount from 1 to 10%, and silicone wax in 1 to 10% (e.g., about 2% or about 2.5%). The waxes provide structure to the cosmetic compositions and in the case of mascara, provide volumizing and lengthening of the eyelash, and are selected, as set forth herein, so that the clarity of the cosmetic base and the gel base are minimally affected. Accordingly, a heightened depth of the color is obtained from the pigments in the composition, as this cosmetic base enhances the darkening provided by the mascara upon application. Further, the low opacity wax component in the cosmetic composition provides volumizing and lengthening effects, while retaining suitable flexibility as a consequence of the reduced wax levels. In further embodiments, the mascara composition of the current invention may also include polyamide resins, such as ethylenediamine/hydrogenated dimer dilinoleate alkyl amide, as further structuring agents, and/or low opacity fillers, and/or low refractive index fillers, as hereinafter described. In some embodiments, the cosmetic composition comprises a gel component. The gel component will typically comprise one or more gelling agents and a solvent or oil that is gelled by the gelling agents. The solvent can be any suitable solvent, either aqueous or non-aqueous, and my include water, lower alcohols, fatty alcohols, esters, hydrocarbon oils, silicone oils, and the like. The gel component typically has a ΔL* value of less than 10. The individual gelling agents may have a ΔL* value of less than 10, or, if more than one gelling agent is used, the aggregate of the gelling agents may have a ΔL* value of less than 7.5 In some embodiments, the gel component (or individual gelling agents) may have a ΔL* value of less than 9, less than 8, less than 7.5, less than 7, less than 6, less than 5, less than 4, less than 3, or less than 2. As used herein the term "substantially transparent" as applied to the gel component means a ΔL* that is 10 or less. In one embodiment, the wax component in said composition consists predominantly of silicone wax and beeswax. In one embodiment, the wax component in said composition consists essentially of silicone wax and beeswax.

In some embodiments, cosmetic compositions are provided comprising a gel component, a wax component, and one or more pigments (e.g., iron oxide and/or carbon black). The gel component may have a ΔL* value of less than 7.5 (e.g., less than 7, less than 6, or less than 5), and may comprise the one or more gellants (e.g., glutamide gellants, polyamide gellants, ester-terminated polyester amides, cellulosics, acrylates, etc.).

The cosmetic compositions comprise a wax component, an oil phase gellant, one or more pigment and/or filler particulates, and an oil capable of forming a gel with the gellants.

The gel component, or the gellant, comprises the glutamide-based gellants, dibutyl lauryl glutamide and/or dibutyl ethylhexanoyl glutamide. In some embodiments the composition may comprise dibutyl lauroyl glutamide, and in further embodiments the composition may comprise both dibutyl lauryl glutamide and dibutyl ethylhexanoyl glutamide. For example, GP-1 as dibutyl lauroyl glutamide and EB-21 as dibutyl ethylhexanoyl glutamide (both manufactured by AJINOMOTO CO., INC.) *etc.,* are suitable. A glutamide compound "consisting essentially of" dibutyl ethylhexanoyl glutamide is intended to mean that the presence of additional glutamide compounds in amounts which would measurably affect the stability and/or viscosity of the fluid are excluded. In one embodiment, the gel component comprises greater than 50% by weight of a glutamide gellant.

In one embodiment, the gellant comprises dibutyl laurolyl glutamide and/or dibutyl ethylhexanoyl glutamide.

In one embodiment, the gel component may comprise a polyamide gellant. In another embodiment, the gel component comprises an ester-terminated polyester amide, such as Sylvaclear C75 (Arizona Chemicals). Sylvaclear C75 and additional suitable gellants and solvents therefore are described in U.S. Patent No. 7,989,002. Also suitable are the gellants and solvents therefore as described in U.S. Patent No.7,682,621.

The composition comprises between 0.1% and 1% of an oil phase gallant. The gellants will typically be present in an amount sufficient to stabilize or to structure the composition. In some specific embodiments, gellants are present in an amount of about 0.3%, about 0.2%, about 0.3%, about 0.4%, about 0.5%, about 0.6%, about 0.7%, about 0.8%, about 0.9%, or about 1.0%, by weight of the composition.

### Particulates

Particulates may include any suitable pigments, lakes, etc. Particulates will typically have a particle size between about 1 nm and about 1 mm. In some embodiments, at least 90% of the volume of particulates has a size (average diameter) greater than about 10 nm. In some embodiments, less than 10% of the total volume of particulates has a size greater than 100 microns.

### Pigments

For purposes of the current invention, "pigments" shall be defined as organic pigments, inorganic pigments, lakes, pearlescent pigments, and or combinations thereof. Typically the compositions will include pigments to impart a desired color or effect. Mascaras of the current invention may include black including various shades as well as additional known colors for mascaras. In certain, embodiments, the color white may be excluded from the colors of mascara available.

Examples of pigments are inorganic pigments, organic pigments, and/or lakes. Exemplary inorganic pigments include, but are not limited to, metal oxides and metal hydroxides such as magnesium oxide, magnesium hydroxide, calcium oxide, calcium hydroxides, aluminum oxide, aluminum hydroxide, iron oxides (α-Fe₂O₃, γ-Fe₂O₃, Fe₃O₄, FeO), red iron oxide, yellow iron oxide, black iron oxide, iron hydroxides, titanium dioxide, titanium lower oxides, zirconium oxides, chromium oxides, chromium hydroxides, manganese oxides, cobalt oxides, cerium oxides, nickel oxides and zinc oxides as well as composite oxides and composite hydroxides such as iron titanate, cobalt titanate and cobalt aluminate. Non-metal oxides also contemplated to be suitable are alumina and silica, ultramarine blue (i.e., sodium aluminum silicate containing sulfur), Prussian blue, manganese violet, bismuth oxychloride, talc, mica, sericite, magnesium carbonate, calcium carbonate, magnesium silicate, aluminum magnesium silicate, silica, titanated mica, iron oxide titanated mica, bismuth oxychloride, and the like. Organic pigments can include, but are not limited to, at least one of carbon black, carmine, phthalocyanine blue and green pigment, diarylide yellow and orange pigments, and azo-type red and yellow pigments such as toluidine red, litho red, naphthol red and brown pigments, and combinations thereof.

Lakes generally refer to a colorant prepared from a water-soluble organic dye, (e.g., D&C or FD&C) which has been precipitated onto an insoluble reactive or absorptive substratum or diluent. The term "D&C" as used herein means drug and cosmetic colorants that are approved for use in drugs and cosmetics by the FDA. The term "FD&C" as used herein means food, drug, and cosmetic colorants which are approved for use in foods, drugs, and cosmetics by the FDA. Certified D&C and FD&C colorants suitable for precipitation onto the insoluble reactive or absorptive stratum of lakes are listed in 21 C.F.R. § 74.101 *et seq.* and include the FD&C colors Blue 1, Blue 2, Green 3, Orange B, Citrus Red 2, Red 3, Red 4, Red 40, Yellow 5, Yellow 6, Blue 1, Blue 2, Orange B, Citrus Red 2, and the D&C colors Blue 4, Blue 9, Green 5, Green 6, Green 8, Orange 4, Orange 5, Orange 10, Orange 11, Red 6, Red 7, Red 17, Red 21, Red 22, Red 27, Red 28, Red 30, Red 31, Red 33, Red 34, Red 36, Red 39, Violet 2, Yellow 7, Yellow 8, Yellow 10, Yellow 11, Blue 4, Blue 6, Green 5, Green 6, Green 8, Orange 4, Orange 5, Orange 10, Orange 11, and so on. Suitable lakes include, without limitation, those of red dyes from the monoazo, disazo, fluoran, xanthene, or indigoid families, such as Red 4, 6, 7, 17, 21, 22, 27, 28, 30, 31, 33, 34, 36, and Red 40; lakes of yellow pyrazole, monoazo, fluoran, xanthene, quinoline, dyes or salt thereof, such as Yellow 5, 6, 7, 8, 10, and 11; lakes of violet dyes including those from the anthroquinone family, such as Violet 2, as well as lakes of orange dyes, including Orange 4, 5, 10, 11, and the like. Suitable lakes of D&C and FD&C dyes are defined in 21 C.F.R. § 82.51.

The pigments may be optionally surface treated to, for example, make the particles more hydrophobic or more dispersible in a vehicle. The surface of the particles may, for example, be covalently or ionically bound to an organic molecule or silicon-based molecule or may be absorbed thereto, or the particle may be physically coated with a layer of material. The surface treatment compound may be attached to the particle through any suitable coupling agent, linker group, or functional group (e.g., silane, ester, ether, etc). The compound may comprise a hydrophobic portion which may be selected from, for example, alkyl, aryl, allyl, vinyl, alkyl-aryl, aryl-alkyl, organosilicone, di-organosilicone, dimethicones, methicones, polyurethanes, silicone-polyurethanes, and fluoro- or perfluoro-derivatives thereof. Other hydrophobic modifiers include, but are not limited, lauroyl lysine, Isopropyl Titanium Triisostearate (ITT), ITT and Dimethicone (ITT/Dimethicone) cross-polymers, ITT and Amino Acid, ITT/Triethoxycaprylylsilane Crosspolymer, waxes (e.g., carnauba), fatty acids (e.g., stearates), HDI/Trimethylol Hexylactone Crosspolymer, PEG-8 Methyl. Ether Triethoxysilane, aloe, jojoba ester, lecithin, perfluoroalcohol phosphate, and Magnesium Myristate (MM). In other embodiments, the pigments or particulates may be surface treated with galactoarabinase or glyceryl rosinate. In another embodiment, the pigments or particulates may be surface treated with Disodium Stearoyl Glutamate (and) Aluminum Dimyristate (and) Triethoxycaprylysilane.

In addition to the foregoing, the compositions according to the invention may comprise additional pigments, and/or pearlescents. Inorganic pigments include without limitation titanium dioxide, zinc oxide, iron oxides, chromium oxide, ferric blue, mica, bismuth oxychloride, and titinated mica; organic pigments include barium, strontium, calcium or aluminum lakes, ultramarines, and carbon black. In certain, embodiments mascaras of the current invention exclude white pigments (e.g., titanium dioxide, zinc oxide, or calcium carbonate).

The pigments may be surface modified with, for example, fluoropolymers, to adjust one or more characteristics of the colorant as described in, for example, U.S. Patent Nos. 6,471,950, 5,482,547, and 4,832,944. Suitable pearling pigments include without limitation bismuth oxychloride, guanine and titanium composite materials containing, as a titanium component, titanium dioxide, titanium lower oxides or titanium oxynitride, as disclosed in U.S. Patent No. 5,340,569. Other suitable pearlescent materials typically are pigments or layers of titanium dioxide on a substrate such as mica, polyethylene terephthalate, bismuth oxychloride, aluminum oxide, calcium borosilicate, synthetic flourophlogopite (synthetic mica), silica, acrylates copolymer, methyl methacrylate, and the like. Interference or pearl pigments may also be included. These are typically comprised of micas layered with about 50 to 300 nm films of TiO₂, Fe₂O₃, or Cr₂O₃ or the like. These include white nacreous materials, such as mica covered with titanium oxide or covered with bismuth oxychloride; and colored nacreous materials, such as titanium mica with iron oxides, titanium mica with ferric blue or chromium oxide, titanium mica with an organic pigment of the aforementioned type.

The pearlescent pigments can be chosen from white pearlescent pigments, such as mica covered with titanium or with bismuth oxychloride, colored pearlescent pigments, such as titanium oxide-coated mica with iron oxides, titanium oxide-coated mica with in particular ferric blue or chromium oxide, or titanium oxide-coated mica with an organic pigment of the abovementioned type, and pearlescent pigments based on bismuth oxychloride. Commercially available pearlescent pigments suitable for the current invention include, but are not limited to, MicaMira (Sandream Enterprises), SynMira (Sandream Enterprises), GlassMira (Sandream Enterprises), Xirona (EMD Performance Chemicals), Timiron (EMD Performance Chemicals), Colorona (EMD Performance Chemicals), Ronastar (EMD Performance Chemicals), RonaFlair (EMD Performance Chemicals), Reflecks (BASF), Duocrome (BASF), and Chione (BASF).

Preferred pigments include Iron Oxides, Black Oxide of Iron, Brown Iron Oxide, Iron Oxide Red 10-34-PC-2045, Pigment Black 11, Pigment Brown 6, Pigment Brown 7, Pigment Red 101, Pigment Red 102, Pigment Yellow 42, Pigment Yellow 43, Red Iron Oxide, Synthetic Iron Oxide, Yellow Iron Oxide, or carbon black. In those embodiments where carbon black is used as a pigment all or a portion thereof may be dispersed in a suitable synthetic wax. The mascara compositions herein are particularly useful in providing an enhanced depth of a dark color to the eyelashes, especially a more pronounced black color to the eyelashes, especially a color having a tristimulus L* value less than about 20, 15 or less, 12.5 or less, 10 or less, or 7.5 or less, as measured using a Konica Minolta CM-2600d Spectrophotometer.

The composition of the present invention comprises less than 15% by weight of one or more pigments and/or fillers. Typically, additional pigments and/or colorants may comprise from 0.1% to 15% of the total composition, from 1% to 12% by weight of the composition, or from 3% to 10% by weight of the composition. In certain embodiments, the composition will contain 1%, 2.5%, 5%, 7.5%, 10%, 12.5%, or 15% pigments. In embodiments incorporating carbon black as a pigment, the amount of carbon black incorporated may be 0.005% to 0.025%, 0.1% to 5%, 0.5% to 4%, and 1% to 3%. When the mascara composition is a mascara emulsion, the pigments are added to the phase in which they are most compatible. For example pigments that have a hydrophobic treatment would be incorporated into the lipophilic phase, while pigments having a hydrophilic treatment would be in the aqueous phase. Pigments with silicone coatings would be incorporated into the silicone phase of a silicone-water emulsion.

In some embodiments, the total amount of particulates present in the composition is less than 25% by weight, or less than 20% by weight, or less than 15% by weight, or less than 12.5% by weight, or less than 10% by weight, or less than 9% by weight, or less than 8% by weight, or less than 7% by weight, or less than 6% by weight, or less than 5% by weight, or less than 4% by weight, or less than 3% by weight, or less than 2% by weight, or less than 1% by weight, or less than 0.5% by weight of the composition. In some embodiments, the compositions are free of particulates.

### Emulsion

The cosmetic compositions may also comprise an emulsion. Non-limiting examples of suitable emulsions include water-in-oil emulsions, oil-in-water emulsions, silicone-in-water emulsions, water-in-silicone emulsions, wax-in-water emulsions, water-oil-water triple emulsions or the like having the appearance of a cream, gel or microemulsions. The emulsion may include an emulsifier, such as a nonionic, anionic or amphoteric surfactant.

The aqueous phase of the emulsion in one embodiment has one or more organic compounds, including humectants (such as butylene glycol, propylene glycol, Methyl gluceth-20, and glycerin); other water-dispersible or water-soluble components including thickeners such as veegum or hydroxyalkyl cellulose; gelling agents, such as high MW polyacrylic acid, i.e. Carbopol 934; and mixtures thereof. In one embodiment, the aqueous phase may include a film forming polymer, for example an acrylate copolymer. In one embodiment, an acrylates copolymer is characterized as having a viscosity of about 25 mPa.s (cps) in a 30% aqueous solution. The emulsion may have one or more emulsifiers capable of emulsifying the various components present in the composition.

The compounds suitable for use in the oil phase include without limitation, vegetable oils; esters including emollient esters, such as octyl palmitate, isopropyl myristate and isopropyl palmitate; ethers such as dicapryl ether; fatty alcohols such as cetyl alcohol, stearyl alcohol and behenyl alcohol; isoparaffins such as isooctane, isododecane and isohexadecane; silicone oils such as dimethicones, cyclic silicones, and polysiloxanes; hydrocarbon oils such as mineral oil, petrolatum, isoeicosane and polyisobutene; and the like. Suitable hydrophobic hydrocarbon oils may be saturated or unsaturated, have an aliphatic character and be straight or branched chained or contain alicyclic or aromatic rings. The oil-containing phase may be composed of a singular oil or mixtures of different oils.

In one embodiment, the oil phase will include an amount of octyldodecanol sufficient to solubilize the amino acid gellants. In one embodiment, the oil phase will include a film forming polymer (e.g., VP hexadecene copolymer). The film former may act to reduce smudging.

Hydrocarbon oils including those having 6-20 carbon atoms may be utilized, and in one embodiment they may have 10-16 carbon atoms. Representative hydrocarbons include decane, dodecane, tetradecane, tridecane, and C₈₋₂₀ isoparaffins. Paraffinic hydrocarbons are available from Exxon under the ISOPARS trademark, and from the Permethyl Corporation. In addition, C₈₋₂₀ paraffinic hydrocarbons such as C₁₂ isoparaffin (isododecane) manufactured by the Permethyl Corporation having the tradename Permethyl 99A™ are also contemplated to be suitable. Various commercially available C₁₆ isoparaffins, such as isohexadecane (having the tradename Permethyl®) are also suitable. Examples of volatile hydrocarbons include polydecanes such as isododecane and isodecane, including for example, Permethyl-99A (Presperse Inc.) and the C₇-C₈ through C₁₂-C₁₅ isoparaffins such as the Isopar Series available from Exxon Chemicals. A representative hydrocarbon solvent is isododecane. Oils that are capable of forming a film with an oil phase gellant may be present in an amount between 0.5% and 20%, or between 1% and 15%, or between 3% and 10%, or between 5% and 8% of the composition.

Non-limiting emulsifiers include emulsifying waxes, emulsifying polyhydric alcohols, polyether polyols, polyethers, mono- or di-ester of polyols, ethylene glycol mono-stearates, glycerin mono-stearates, glycerin di-stearates, silicone-containing emulsifiers, soya sterols, fatty alcohols such as cetyl alcohol, acrylates, fatty acids such as stearic acid, fatty acid salts, and mixtures thereof. Emulsifiers may include soya sterol, cetyl alcohol, stearic acid, emulsifying wax, acrylates, silicone containing emulsifiers and mixtures thereof. Other specific emulsifiers that can be used in the composition of the present invention include, but are not limited to, one or more of the following: C₁₀₋₃₀ alkyl acrylate crosspolymer; Dimethicone PEG-7 isostearate, acrylamide copolymer; mineral oil; sorbitan esters; polyglyceryl-3-diisostearate; sorbitan monostearate, sorbitan tristearate, sorbitan sesquioleate, sorbitan monooleate; glycerol esters such as glycerol monostearate and glycerol monooleate; polyoxyethylene phenols such as polyoxyethylene octyl phenol and polyoxyethylene nonyl phenol; polyoxyethylene ethers such as polyoxyethylene cetyl ether and polyoxyethylene stearyl ether; polyoxyethylene glycol esters; polyoxyethylene sorbitan esters; dimethicone copolyols; polyglyceryl esters such as polyglyceryl-3-diisostearate; glyceryl laurate; Steareth-2, Steareth-10, and Steareth-20, to name a few. Additional emulsifiers are provided in the INCI Ingredient Dictionary and Handbook 11th Edition 2006.

These emulsifiers typically will be present in the composition in an amount from 0.001% to 10% by weight, in particular in an amount from 0.01% to 5% by weight, and in one embodiment, from 0.1% to 3% by weight.

The oil phase may comprise one or more volatile and/or non-volatile silicone oils. Volatile silicones include cyclic and linear volatile dimethylsiloxane silicones. In one embodiment, the volatile silicones may include cyclodimethicones, including tetramer (D₄), pentamer (D₅), and hexamer (D₆) cyclomethicones, or mixtures thereof. Particular mention may be made of the volatile cyclomethicone-hexamethyl cyclotrisiloxane, octamethyl-cyclotetrasiloxane, and decamethyl-cyclopentasiloxane. Suitable dimethicones are available from Dow Corning under the name Dow Corning 200® Fluid and have viscosities ranging from 0.65 to 600,000 mm.s⁻¹ (centistokes) or higher. Suitable non-polar, volatile liquid silicone oils are disclosed in U.S. Pat. No. 4,781,917. Additional volatile silicones materials are described in Todd et al., "Volatile Silicone Fluids for Cosmetics", Cosmetics and Toiletries, 91:27-32 (1976). Linear volatile silicones generally have a viscosity of less than 5 mm².s⁻¹ (centistokes) at 25° C, whereas the cyclic silicones have viscosities of less than 10 mm².s⁻¹ (centistokes) at 25° C. Examples of volatile silicones of varying viscosities include Dow Corning 200, Dow Corning 244, Dow Corning 245, Dow Corning 344, and Dow Corning 345, (Dow Corning Corp.); SF-1204 and SF-1202 Silicone Fluids (G.E. Silicones), GE 7207 and 7158 (General Electric Co.); and SWS-03314 (SWS Silicones Corp.). Linear, volatile silicones include low molecular weight polydimethylsiloxane compounds such as hexamethyldisiloxane, octamethyltrisiloxane, decamethyltetrasiloxane, and dodecamethylpentasiloxane, to name a few.

Non-volatile silicone oils will typically comprise polyalkylsiloxanes, polyarylsiloxanes, polyalkylarylsiloxanes, or mixtures thereof. Polydimethylsiloxanes are non-volatile silicone oils. The non-volatile silicone oils will typically have a viscosity from 10 to 60,000 mm².s⁻¹ (centistokes) at 25° C, in one embodiment between 10 and 10,000 mm².s⁻¹ (centistokes), and in one embodiment still between 10 and 500 mm².s⁻¹ (centistokes); and a boiling point greater than 250° C at atmospheric pressure. Non limiting examples include dimethyl polysiloxane (dimethicone), phenyl trimethicone, and diphenyldimethicone. The volatile and non-volatile silicone oils may optionally be substituted with various functional groups such as alkyl, aryl, amine groups, vinyl, hydroxyl, haloalkyl groups, alkylaryl groups, and acrylate groups, to name a few.

The water-in-silicone emulsion may be emulsified with a nonionic surfactant (emulsifier) such as, for example, polydiorganosiloxane-polyoxyalkylene block copolymers, including those described in U.S. Pat. No. 4,122,029. These emulsifiers generally comprise a polydiorganosiloxane backbone, typically polydimethylsiloxane, having side chains comprising ―(EO)ₘ― and/or —(PO)ₙ— groups, where EO is ethyleneoxy and PO is 1,2-propyleneoxy, the side chains being typically capped or terminated with hydrogen or lower alkyl groups (e.g., C₁₋₆, typically C₁₋₃). Other suitable water-in-silicone emulsifiers are disclosed in U.S. Pat. No. 6,685,952. Commercially available water-in-silicone emulsifiers include those available from Dow Corning under the trade designations 3225C and 5225C FORMULATION AID; SILICONE SF-1528 available from General Electric; ABIL EM 90 and EM 97, available from Goldschmidt Chemical Corporation (Hopewell, Va.); and the SILWET series of emulsifiers sold by OSI Specialties (Danbury, Conn.).

Examples of water-in-silicone emulsifiers include, but are not limited to, dimethicone PEG 10/15 crosspolymer, dimethicone copolyol, cetyl dimethicone copolyol, PEG-15 lauryl dimethicone crosspolymer, laurylmethicone crosspolymer, cyclomethicone and dimethicone copolyol, dimethicone copolyol (and) caprylic/capric triglycerides, polyglyceryl-4 isostearate (and) cetyl dimethicone copolyol (and) hexyl laurate, and dimethicone copolyol (and) cyclopentasiloxane. In one embodiment examples of water-in-silicone emulsifiers include, without limitation, PEG/PPG-18/18 dimethicone (trade name 5225C, Dow Corning), PEG/PPG-19/19 dimethicone (trade name BY25-337, Dow Corning), Cetyl PEG/PPG-10/1 dimethicone (trade name Abil EM-90, Goldschmidt Chemical Corporation), PEG-12 dimethicone (trade name SF 1288, General Electric), lauryl PEG/PPG-18/18 methicone (trade name 5200 FORMULATION AID, Dow Corning), PEG-12 dimethicone crosspolymer (trade name 9010 and 9011 silicone elastomer blend, Dow Corning), PEG-10 dimethicone crosspolymer (trade name KSG-20, Shin-Etsu), dimethicone PEG-10/15 crosspolymer (trade name KSG-210, Shin-Etsu), and dimethicone PEG-7 isostearate.

The emulsifiers typically will be present in the composition in an amount effective to disperse the discontinuous phase into the continuous phase, typically from 0.001% to 10% by weight, in another embodiment in an amount from 0.01% to 5% by weight, and in a further embodiment in an amount below 1% by weight.

The aqueous phase of the emulsion may include one or more volatile solvents, including lower alcohols, such as ethanol, isopropanol, and the like. The volatile solvent may also be a cosmetically acceptable ester such as butyl acetate or ethyl acetate; ketones such as acetone or ethyl methyl ketone; or the like. The volatile solvents are generally present in an amount of 25% or less by weight of the composition. In other embodiments the volatile solvent is present in an amount of less than 15%, less than 10%, or less than 5% by weight of the composition. In another embodiment the compositions do not contain a volatile solvent.

The non-aqueous phase will typically comprise from 10% to 90%, 30% to 80%, or from 50% to 70% by weight, based on the total weight of the emulsion, and the aqueous phase will typically comprise from 10% to 90%, 30% to 80%, or from 40% to 70% by weight of the total emulsion. In one embodiment of the invention the mascara composition is a water-in-silicone emulsion in which the aqueous phase is from 20% to 60% by weight of the total composition and the non-aqueous silicone phase is from 40% to 80% by weight of the total composition. In one embodiment of the invention the mascara composition is a water-in-oil or oil-in-water emulsion in which the aqueous phase is about 60% by weight of the total composition and the non-aqueous oil phase is about 40% by weight of the total composition.

### Anhydrous Vehicle

Cosmetic compositions comprising an anhydrous vehicle include without limitation, vegetable oils; esters including emollient esters, such as octyl palmitate, isopropyl myristate and isopropyl palmitate; ethers such as dicapryl ether; fatty alcohols such as cetyl alcohol, stearyl alcohol octyldodecanol and behenyl alcohol; isoparaffins such as isooctane, isododecane and isohexadecane; silicone oils such as dimethicones, cyclic silicones, and polysiloxanes; hydrocarbon oils such as mineral oil, petrolatum, isoeicosane and polyisobutene; and the like. Suitable hydrophobic hydrocarbon oils may be saturated or unsaturated, have an aliphatic character and be straight or branched chained or contain alicyclic or aromatic rings.

Hydrocarbon oils including those having 6-20 carbon atoms may be utilized, and in one embodiment they may have 10-16 carbon atoms. Representative hydrocarbons include decane, dodecane, tetradecane, tridecane, and C₈₋₂₀ isoparaffins. Paraffinic hydrocarbons are available from Exxon under the ISOPARS trademark, and from the Permethyl Corporation. In addition, C₈₋₂₀ paraffinic hydrocarbons such as C₁₂ isoparaffin (isododecane) manufactured by the Permethyl Corporation having the tradename Permethyl 99A™ are also contemplated to be suitable. Various commercially available C₁₆ isoparaffins, such as isohexadecane (having the tradename Permethyl®) are also suitable. Examples of volatile hydrocarbons include polydecanes such as isododecane and isodecane, including for example, Permethyl-99A (Presperse Inc.) and the C₇-C₈ through C₁₂-C₁₅ isoparaffins such as the Isopar series available from Exxon Chemicals. A representative hydrocarbon solvent is isododecane.

The oil phase may comprise one or more volatile and/or non-volatile silicone oils. Volatile silicones include cyclic and linear volatile dimethylsiloxane silicones. In one embodiment, the volatile silicones may include cyclodimethicones, including tetramer (D₄), pentamer (D₅), and hexamer (D₆) cyclomethicones, or mixtures thereof. Particular mention may be made of the volatile cyclomethicone-hexamethyl cyclotrisiloxane, octamethyl-cyclotetrasiloxane, and decamethyl-cyclopentasiloxane. Suitable dimethicones are available from Dow Corning under the name Dow Corning 200® Fluid and have viscosities ranging from 0.65 to 600,000 centistokes or higher. Suitable non-polar, volatile liquid silicone oils are disclosed in U.S. Pat. No. 4,781,917. Additional volatile silicones materials are described in Todd et al., "Volatile Silicone Fluids for Cosmetics", Cosmetics and Toiletries, 91:27-32 (1976). Linear volatile silicones generally have a viscosity of less than about 5 mm.s⁻¹ (centistokes) at 25° C, whereas the cyclic silicones have viscosities of less than about 10 mm.s⁻¹ (centistokes) at 25° C Examples of volatile silicones of varying viscosities include Dow Corning 200, Dow Corning 244, Dow Corning 245, Dow Corning 344, and Dow Corning 345, (Dow Corning Corp.); SF-1204 and SF-1202 Silicone Fluids (G.E. Silicones), GE 7207 and 7158 (General Electric Co.); and SWS-03314 (SWS Silicones Corp.). Linear, volatile silicones include low molecular weight polydimethylsiloxane compounds such as hexamethyldisiloxane, octamethyltrisiloxane, decamethyltetrasiloxane, and dodecamethylpentasiloxane, to name a few.

Non-volatile silicone oils will typically comprise polyalkylsiloxanes, polyarylsiloxanes, polyalkylarylsiloxanes, or mixtures thereof. Polydimethylsiloxanes are non-volatile silicone oils. The non-volatile silicone oils will typically have a viscosity from 10 to 60,000 mm.s⁻¹ (centistokes) at 25° C, in one embodiment between 10 and 10,000 mm.s⁻¹ (centistokes), and in one embodiment still between 10 and 500 mm.s⁻¹ (centistokes); and a boiling point greater than 250° C at atmospheric pressure. Non limiting examples include dimethyl polysiloxane (dimethicone), phenyl trimethicone, and diphenyldimethicone. The volatile and non-volatile silicone oils may optionally be substituted with various functional groups such as alkyl, aryl, amine groups, vinyl, hydroxyl, haloalkyl groups, alkylaryl groups, and acrylate groups, to name a few.

The anhydrous vehicle may comprise a non-ionic unsaturated fatty alcohol which can dissolve the glutamide based gellants. One or more of the non-ionic unsaturated fatty alcohols useful for dissolution includes but is not limited to a non-ionic mono- or poly-unsaturated fatty alcohol. Non-limiting examples of useful non-ionic unsaturated fatty alcohols of the disclosure include oleyl alcohols, octyldodecanols, 2-butyloctanals, 2-hexyldecanols, and 2-undecylpentadecanols. A particular embodiment is directed to oleyl alcohol. Oleyl alcohol examples include, but are not limited to octadecenol.. The non-ionic unsaturated fatty alcohol of the inventive composition may be present in an amount from 0.1% to 30% by weight of the total composition. Other embodiments are directed to an amount of 15% to 30%, and further, 16% to 25.5% by weight of the total composition. The anhydrous vehicle may comprise up to 75% of the composition, typically 10% to 60%, usually 20% to 50%, and especially 25% to 40% by weight of the mascara composition.

The compositions herein are particularly useful in providing an enhanced depth of a dark color, for example, to the eyelashes, especially, for example, a more pronounced black color to the eyelashes. The overall opacity of the cosmetic base of the mascara composition of the current invention is sufficiently low to permit the desired enhancement in the depth of color, i.e., to provide the deep dark color consumers prefer. In the L* a* b* color space (also known as CIELAB), L* indicates lightness and a* and b* are the color directions. L* is measured from 0 (black) to 100 (white). When the inventive formulations are dark or especially black, the black pigments in the formulations have increased light absorption and decreased light reflection, that is, a decreased L* value (i.e., a more intense black). In one embodiment, the mascara composition has a tristimulus L* value of less than 20. In other embodiments L* is 15 or less, 12.5 or less, 10 or less, or 7.5 or less, as measured using a Konica Minolta CM-2600d Spectrophotometer. L* is measured by measuring L* values on a drawdown film (as hereinafter described) of mascara on a black Leneta card using a Konica Minolta CM-2600d hand-held spectrophotometer.

The mascara compositions of the current invention may have a consistency of a liquid and/or viscous liquid. The hardness of the mascara may be measured by penetrating a probe into the composition. In particular, a texture analyzer (for example TA-XT2i from Rheo) equipped with a 2 mm needle probe may be used. The texture analyzer may be set to: Measurement Mode: Force in Compression; Test Speed: 1.0 mm/s; Distance: 5 mm; and Trigger Force: 5 g. The mascara compositions of the current invention may have a penetrating force of less than 15 g and in other embodiments the penetrating force may be less than 10 g. The hardness value may be between 1 g and 15 g.

Additionally, the compositions of the current invention may exhibit a viscosity between 250,000 mPa.s (centipoise) and 2,000,000 mPa.s (centipoise), in another embodiment between 500,000 mPa.s (centipoise) and 1,750,000 mPa.s (centipoise); and 750,000 mPa.s (centipoise) and about 1,500,000 mPa.s (centipoise). The viscosity of the composition may be determined by using a Brookfield DV-E viscometer rotating at 4 rpms with a T-bar E spindle, at 25°C. In one embodiment, the composition is in the form of a liquid having a viscosity between 250,000 mPa.s (centipoise) and 2,000,000 mPa.s (centipoise).

The cosmetic compositions of the invention may provide a high payoff. Payoff describes the ability of a compound to impart a film onto a surface, such as a human integument (as measured according to Example 6). In one embodiment, the cosmetic composition provides a payoff value that is greater than the payoff value of an otherwise identical composition that lacks the gel component or the wax component. In another embodiment, the cosmetic composition provides a higher payoff compared to a traditional, high-opacity wax based cosmetic, such as a high-opacity wax based mascara.

The compositions of the invention also may provide excellent multi-angle chroma and color travel, as measured according to Example 5. In one embodiment, the cosmetic composition has superior color travel compared to the color travel of an otherwise identical composition that lacks the gel component or the wax component. In one embodiment, the cosmetic composition has superior chroma, and multi-angle chroma compared to the chroma and multi-angle chroma of an otherwise identical composition that lacks the gel component or the wax component. In another embodiment, the cosmetic composition has superior color intensity and/or hue, as compared to the color intensity and/or hue of an otherwise identical composition that lacks the gel component or the wax component. In another embodiment, the cosmetic composition has superior color travel, multi-angle chroma, color intensity, and/or hue, as compared to those features in a traditional, high-opacity wax based cosmetic, such as a high-opacity wax based mascara.

In certain embodiments of the invention, the cosmetic composition is substantially free of alkyl dimethicone. "Substantially free," in this context means that there is less than 1%, in some embodiments less than 0.5%, in further embodiments less than 0.05%, and in yet further embodiments 0% of alkyl dimethicone. In other embodiments, the compositions include alkyl dimethicone (e.g., stearyl dimethicone). In one embodiment, the amount of alkyl dimethicone (e.g., stearyl dimethicone) is between 1% and 5% by weight, or between 1.5% and 3.5% by weight.

In an additional embodiment of the current invention, a polyamide resin may provide additional structural integrity to the gel base. Polyamide resins are high molecular weight polymers which feature amide linkages along the molecular chain. These polymers contain monomers of amides joined by peptide bonds. They can occur both naturally and artificially. Such polymers are made through step growth polymerization or solid phase synthesis. In some cases, examples of polyamide resins are nylons and aramids. Due to their extreme durability and strength, polyamide resins are typically utilized in textiles, plastics and various automotive applications. In the composition of the present invention the polyamide resin also provides a degree of gloss or shine to the composition and adhesion to the target substrate.

In one preferred embodiment, the polyamide resin comprises Ethylenediamine Hydrogenated Dimer Dilinoleate Copolymer Bis-Di-C14-18 Alkyl Amide, however the invention is not limited to this polyamide resin. One skilled in the art will be able to select suitable polyamide resins and many suitable polymers are disclosed in the CTFA Handbook, 12'h Ed. 2008. These include, without limitation, Polyamide-1, Polyamide-2, Polyamide-3, Ethylenediamine/Dimer Tallate Copolymer Bis-Hydrogenated Tallow Amide, Ethylenediamine/Stearyl Dimer Dilinoleate Copolymer, Ethylenediamine/Stearyl Dimer Tallate Copolymer, etc.

A particular embodiment of the present disclosure is directed to a polyamide resin, or a combination of compatible polyamide resins, in an amount ranging from 0.1% to 25% by weight of the total composition, 5% to 20% by weight, and 7% to 15%. A particularly preferred polyamide resin is Ethylenediamine Hydrogenated Dimer Dilinoleate Copolymer Bis-Di -Cl4-18 Alkyl Amide. In other embodiments, the compositions are free of such additional gellants, including polyamides.

In yet a further embodiment of the cosmetic compositions of the current invention, various low opacity fillers (the low opacity filler component) determined in accordance with the method denoted in Example 1 below, may be incorporated to enhance the dry time and volume of the composition. Fillers suitable for use in the current composition will exhibit a ΔL* value that is less than 8 individually and/or in combination. In other embodiments the ΔL* value of the fillers, singly or in combination, is 6 or less, or 4 or less, or 2 or less, or 1 or less. For clarity, in certain embodiments the filler component can be a combination of one or more individual fillers having a ΔL* value less than 8 with one or more waxes individually having a ΔL* value of 8 or greater that in aggregate so long as the combination of fillers (i.e., the filler component) exhibits a ΔL* value less than 8. In one embodiment the filler component does not contain an individual filler having a ΔL* value of 8 or greater. In other embodiments the filler component does not contain more than 15%, or more than 10%, or more than 5% of a filler whose ΔL* value is 8 or greater, by weight of the filler component. Suitable fillers having a ΔL* value of less than 8 may include, but are not limited to, barium sulfate (e.g., Blanc Fixe XR-HN from Sachtleben Chemie), sericite (e.g., Sericite PHN from Horie Kako), nylon powder, extra fine (e.g., Orgasol 2002 Exd Nat Cos from Arkema or Anbybes from SH Energy & Chemicals, talc Italian (e.g., Supra H USP from Luzenac America), solid glass microspheres (e.g., Prizmalite™ P2011 SL from Prizmalite Industries, Inc.), and combinations thereof. In particular, in certain embodiments solid glass microspheres may be used as inventors have noted that the use of the spheres provides enhanced depth of color and may provide a cumulative and/or synergistic enhancement in depth of color when used in conjunction with the inventive mascara of the current invention.

The aggregate amount of the filler component is not particularly restricted. Typically, the filler component, if present, will collectively comprise from 0.1% to 10% of the total composition, 0.5% to 7% by weight of the composition, or 1% to 5% by weight of the composition. In certain embodiments the compositions will contain 0.75%, 1.0%, 2.0%, 3.0%, 4.0%, 5.0%, or 7.5% of the filler component.

The cosmetic composition may also contain additional materials such as at least one film-forming agent. The film-forming polymer improves the wear of the composition, and can confer transfer-resistance to the make-up product. The film-forming agent may be any which is cosmetically acceptable for use around the eye. Examples include polymers such as polyethylene polymers, PVP, copolymers of PVP, ethylene vinyl acetate, dimethicone gum, C1-C6 alkyl (meth)acrylate polymer, polyacrylates, polymethacrylates, cellulose polymers, and resins such as trimethylsiloxysilicate. The film former may be used in an amount of from 0.1% to 50%, more preferably from 1 to 30%. The compositions may comprise 0.1% to 50%, 0.5% to 40%, 1% to 30%, 2% to 30%, 1% to 20%, 2% to 20%, 2% to 15%, abot 2% to 10%, 2% to 5%, 5% to 20%, 10% to 20%, 15% to 20%, 10% to 40%, 15% to 35%, or 20% to 30%, relative to the total weight of the composition, of one or more film-forming polymers.

The composition may comprise additional anhydrous compounds including without limitation, vegetable oils; esters such as octyl palmitate, isopropyl myristate, and isopropyl palmitate; ethers such as dicapryl ether; fatty alcohols such as cetyl alcohol, stearyl alcohol and behenyl alcohol; isoparaffins such as isooctane, isododecane, and isohexadecane; silicone oils such as dimethicones, cyclic silicones, and polysiloxanes; hydrocarbon oils such as mineral oil, petrolatum, isoeicosane and polyisobutene; and the like. Suitable hydrophobic hydrocarbon oils may be saturated or unsaturated, have an aliphatic character and be straight or branched chained or contain alicyclic or aromatic rings. The anhydrous vehicle may be composed of a singular oil or mixtures of different oils.

Hydrocarbon oils including those having 6-20 carbon atoms may be utilized, in one embodiment having more preferably 10-16 carbon atoms. Representative hydrocarbons include decane, dodecane, tetradecane, tridecane, and C₈₋₂₀ isoparaffins. Paraffinic hydrocarbons are available from Exxon under the ISOPARS trademark, and from the Permethyl Corporation. In addition, C₈₋₂₀ paraffinic hydrocarbons such as C₁₂ isoparaffin (isododecane) manufactured by the Permethyl Corporation having the tradename Permethyl 99A™ are also contemplated to be suitable. Various commercially available C₁₆ isoparaffins, such as isohexadecane (having the tradename Permethyl®) are also suitable. Examples of preferred volatile hydrocarbons include polydecanes such as isododecane and isodecane, including for example, Permethyl-99A (Presperse Inc.) and the C₇-C₈ through C₁₂-C₁₅ isoparaffins such as the Isopar Series available from Exxon Chemicals. A representative hydrocarbon solvent is isododecane.

The anhydrous vehicle may comprise one or more volatile and/or non-volatile silicone oils. Volatile silicones include cyclic and linear volatile dimethylsiloxane silicones. In one embodiment, the volatile silicones may include cyclodimethicones, including tetramer (D4), pentamer (D5), and hexamer (D6) cyclomethicones, or mixtures thereof. Particular mention may be made of the volatile cyclomethicone-hexamethyl cyclotrisiloxane, octamethyl-cyclotetrasiloxane, and decamethyl-cyclopentasiloxane. Suitable dimethicones are available from Dow Corning under the name Dow Corning 200® Fluid and have viscosities ranging from 0.65 to 600,000 mm².s⁻¹ (centistokes) or higher. Suitable non-polar, volatile liquid silicone oils are disclosed in U.S. Pat. No. 4,781,917. Additional volatile silicone materials are described in Todd et a/., "Volatile Silicone Fluids for Cosmetics", Cosmetics and Toiletries, 91:27-32 (1976). Linear volatile silicones generally have a viscosity of less than 5 mm².s⁻¹ (centistokes) at 25° C, whereas the cyclic silicones have viscosities of less than 10 mm².s⁻¹ (centistokes) at 25° C Examples of volatile silicones of varying viscosities include Dow Corning 200, Dow Corning 244, Dow Corning 245, Dow Corning 344, and Dow Corning 345, (Dow Corning Corp.); SF-1204 and SF-1202 Silicone Fluids (G.E. Silicones), GE 7207 and 7158 (General Electric Co.); and SWS-03314 (SWS Silicones Corp.). Linear, volatile silicones include low molecular weight polydimethylsiloxane compounds such as hexamethyldisiloxane, octamethyltrisiloxane, decamethyltetrasiloxane, and dodecamethylpentasiloxane.

Non-volatile silicone oils will typically comprise polyalkylsiloxanes, polyarylsiloxanes, polyalkylarylsiloxanes, or mixtures thereof. Polydimethylsiloxanes are preferred non-volatile silicone oils. The non-volatile silicone oils will typically have a viscosity from 10 to 60,000 mm².s⁻¹ (centistokes) at 25° C, preferably between 10 and 10,000 mm².s⁻¹ (centistokes), and more preferred still between 10 and 500 mm².s⁻¹ (centistokes); and a boiling point greater than 250° C at atmospheric pressure. Non-limiting examples include dimethyl polysiloxane (dimethicone), phenyl trimethicone, and diphenyldimethicone. The volatile and non-volatile silicone oils may optionally be substituted with various functional groups such as alkyl, aryl, amine groups, vinyl, hydroxyl, haloalkyl groups, alkylaryl groups, and acrylate groups, to name a few.

Viscosifying agents such as gellants may also be used. Examples include bentone, triglycerides, aluminum stearate, C18-C36 acid glycol esters, glyceryl tribehenate, glycerol monostearate, alginates, carbomers, celluloses, gums, carageenans, starches or silicates.

Compounds commonly used in the cosmetic arts for preventing or reducing fungal, bacterial, or microorganismal growth may be also added to the composition of the disclosure. By including these compounds, the shelf life of the composition is lengthened. These anti-fungal and anti-microorganisms include but are not limited to methyl paraben, butyl paraben, sodium dehydroacetate, *etc.* The amounts of these ingredients that may be used within the inventive composition effectively reduce fungal, bacterial, and/or microorganismal growth without negatively affecting the components of the inventive composition or its desired effects.

The compositions of the invention may optionally comprise other active and inactive ingredients typically associated with the intended cosmetic or personal care products. Suitable other ingredients include, but are not limited to, amino acids, antioxidants, conditioners, chelating agents, colorants, emollients, emulsifiers, excipients, fillers, fragrances, gelling agents, humectants, minerals, moisturizers, photostabilizing agents (e.g., UV absorbers), sunscreens, preservatives, stabilizers, staining agents, surfactants, viscosity and/or rheology modifiers, vitamins, waxes and mixtures thereof. Collectively, all such additional components may comprise less than about 5% by weight of the composition.

All ingredients useful herein may be categorized or described by their postulated mode of action. However, it is to be understood that the ingredients can, in some instances, provide more than one cosmetic and/or therapeutic benefit or operate via more than one mode of action. Therefore, classifications herein are made for the sake of convenience and are not intended to limit an ingredient to the particularly stated application or applications listed.

The composition of the invention should be cosmetically or dermatologically acceptable, i.e., it should contain a non-toxic physiologically acceptable medium and should be able to be applied to the eyelashes of human beings. For the purposes of the invention, the expression "cosmetically acceptable" means a composition of pleasant appearance, odor, feel and taste.

The compositions (e.g., mascara compositions) applied to the keratin fibers comprise a wax component (e.g., one or more low opacity waxes), an oil phase gellant, one or more pigments and/or fillers, and an oil capable of forming a gel with the gellants. The low opacity wax component may comprise one or more waxes, and will typically be characterized by a low opacity, such as a ΔL* value of less than 8 (e.g., less than 7, less than 6, or less than 5). Each wax, individually, may also have a ΔL* value of less than 8 (e.g., less than 7, less than 6, or less than 5). The wax component may comprise between 7.5% and 40% by weight of the weight of the entire composition (e.g., 7.5% to 15% by weight). The wax component may comprise, for example, silicone wax, or it may be predominantly comprised of silicone wax, or it may consist essentially of silicone wax, or it may consist of silicone wax. The wax component may consist predominantly of, or consist essentially of, a combination of silicone wax and beeswax. The wax component comprises one or more of carnauba wax, beeswax, Ozokerite, and silicone wax. The gellant is an oil phase gellant comprising dibutyl laurolyl glutamide and/or dibutyl ethylhexanoyl glutamide in an amount between 0.1 and 1%. The fillers and/or pigments, including, for example, iron oxide black and/or carbon black), may be present in an amount less than 15%, less than 10%, less than 7%, less than 6%, less than 5%, less than 4%, less than 3%, less than 2%, less than 1%, or less than 0.5% by weight). In some embodiments, the pigment is not a white pigment (e.g., it is not zinc oxide, calcium carbonate, or titanium dioxide). The oil (e.g., a hydrocarbon, including fatty alcohols such as octyldodoecanol) is capable of forming a gel with the oil phase gellant, and is present in an amount between 1% and 10%, e.g. between 3% and 10%, or between 5% and 8%.

It should be noted that although reference is made throughout to mascara compositions, the inventive compositions and methods are applicable to any kind of cosmetic composition, including, for example, lipstick, lip color, lip gloss, nail polish, foundation, eye liner, and the like, as well as to any suitable personal care product, such as day creams or lotions, night creams or lotions, sunscreen lotions, sunscreen creams, sunscreen sprays or oils and other SPF products, moisturizers, salves, ointments, gels, body milks, artificial tanning compositions, facial masks, depilatories, shampoos, conditioners, hair masks, and the like.

Any of the compositions and ingredients therefor disclosed in U.S. Provisional Patent Application Serial Nos. 61/789,975 and 61/790,104, both filed on March 15, 2013, and in PCT/US14/27609 and PCT/US14/27692, both filed on March 14, 2014, are contemplated to be suitable for practice of the present invention.

Methods for styling keratin fibers are provided. Any keratin fibers may be used, such as, for example, eyelashes, or hair on the head (scalp). The methods comprise applying to a keratin fiber a composition of the invention to form a film on at least a portion of surfaces of the fibers (e.g., along at least a portion, or along a substantial portion, or along a majority of, or along substantially the entire length of the fiber). The film is allowed to dry (i.e., partial or complete evaporation of volatile solvents) to form a moldable film on the keratin fibers, and a user may apply a force to mold the treated fiber into a desired configuration.

As used herein, the term "moldable" refers to a dry film with less than 100% resilience when displaced 2.0 mm by a compressive force at 25°C. In some embodiments, the dry films have a resilience of less than 15%, or less than 12%, or less than 10%, or less than 9%, or less than 8%, or less than 7%, or less than 6%, or less than 5%, or less than 4%, or less than 3%, or less than 2%, or less than 1% when displaced 2.0 mm by a compressive force at 25°C. Resilience refers to the percentage of the compression that recovers when a dry film is subjected to a resilience test as described in Example 8. For example, resilience may be characterized based on a test that displaces a 1-mm thick dry film 2 mm by a compression force at 25°C. The test may be performed on any suitable instrument, such as, for example, the Texture Analyzer described in Example 8.

After the force is removed, the keratin fibers remain in the desired configuration due to the fact that the film behaves substantially non-elastically, or not completely elastically. The keratin fibers may subsequently be re-molded into any number of additional configurations upon the application of subsequent forces, and after each of those forces is removed, the keratin fibers remain in each desired configuration. For example, the keratin fiber may be re-molded into a second desired configuration by applying a second force to the fibers. After the second force is removed, the keratin fibers remain in the second desired configuration.

The keratin fibers may be molded into any desired configuration, such as, for example, curled, straight, crimped, etc., by applying any suitable force to the fibers. The force may include any pressure applied to the fibers, such as, for example, by pressing, brushing, crimping, bending, twirling, squeezing, and so on, either with the use of one's fingers, or with any suitable instrument (e.g., a lash curler or crimper). In some embodiments, the step or steps of molding take place in the absence of added heat.

By way of illustration, after a film of a mascara of the invention has been applied to the eye lashes and dried, the lashes can be molded into a curled configuration and remain curled after the force is removed. Subsequently, the eye lashes may be re-molded to a straightened configuration when a subsequent force is applied, and the lashes will remain straightened until the additional force is removed.

The compositions of the invention may be useful in a method for coloring a human integument, including keratin fibers, comprising applying to the human integument a composition of the invention to form a film thereon. A human integument may include skin, lips, nails, hair, and other keratinous surfaces. As used herein, the term "keratinous surface" refers to keratin-containing portions of the human integumentary system, which includes, but is not limited to, skin, lips, hair (including hair of the scalp, eyelashes, eyebrows, facial hair, and body hair such as hair of the arms, legs, etc.), and nails (toenails, fingernails, cuticles, etc.) of mammalians, preferably humans.

The cosmetic compositions may impart color (e.g., black color) to the human integument (e.g., eyelashes). In another embodiment, the cosmetic compositions impart high chroma to the human integument (e.g., eyelashes), and methods are provided for imparting high chroma to a human integument comprising applying to the human integument a composition according to the invention.

A person skilled in the art will take care to select the optional additives and/or the amount thereof such that the advantageous properties of the composition according to the invention are not, or are not substantially, adversely affected by the envisaged addition. It is further understood that the other cosmetic ingredients and adjuvants introduced into the composition must be of a kind and quantity that are not detrimental to the advantageous effect which is sought herein according to the invention.

The following examples describe specific aspects of the mascara of the present invention to illustrate the invention and provide a description for those skilled in the art. The Examples should not be construed as limiting the invention as the examples merely provide specific methodology useful in the understanding and practice of the invention and its various aspects.

### EXAMPLES

### EXAMPLE 1: Opacity Test Method

The opacity of fillers and waxes, and therefore their impact on the depth of color in a mascara composition of the current invention was determined as follows. A cosmetic base in accordance with the invention as set forth in Table 1 below was prepared for use as a negative control and used to determine the suitability of various waxes and fillers.

**Table 1**

| **Cosmetic Base** | |
|---|---|
| Dibutyl Ethylhexanoyl Glutamide | 1.19% |
| Dibutyl Lauroyl Glutamide | 1.785% |
| Ethylenediamine/Hydrogenated Dimer | 19.05% |
| Dilinoleate Alkyl Amide | |
| Octyldodecanol | 77.975% |

Various gels incorporating waxes and fillers to be evaluated were prepared by replacing 10% of the octyldodecanol in the gelbase. Once prepared, each sample was drawn down (3 mL) on black Leneta cards and allowed to dry for 2 hours. Once dried, five L*, a*, b* readings were calculated per sample using a Konica Minolta CM-2600d spectrophotometer and the data averages were calculated. In one embodiment, any wax or filler component that alone or in the aggregate has a ΔL greater than 8 relative to the control is considered opaque and could interfere with the depth of color within the mascara of the current invention.

**Table 2: Waxes and Fillers**

| **Waxes** | **ΔL** |
|---|---|
| Paraffin Wax | 13.06 |
| Paraffin Wax High Penetration | 12.75 |
| Carnauba Wax | -0.578 |
| Beeswax | 0.608 |
| Beeswax Bleached | 1.026 |
| Ozokerite | -0.346 |
| Kahlwax 7307 | 0.37 |
| Silwax CR M2 | -0.742 |
| Silwax 5022 | -0.712 |
| | |

| **Fillers** | **ΔL** |
|---|---|
| Barium Sulfate | 1.16 |
| Prizmalite™ microspheres | 2.05 |
| POMP 605 | 8.39 |
| Sericite | 7.99 |
| Talc Italian | 1.30 |
| Nylon Powder | 16.19 |

### Example 2: Exemplary Comparative Mascara Formulation

A. A comparative mascara emulsion composition is provided in Table 3.

**Table 3**

| INCI name/description | Wt. % |
|---|---|
| **OIL PHASE** | |
| Cyclomethicone | 20-60 |
| PEG-12 Dimethicone | 1-10 |
| Silicone Fluid | 1-10 |
| Film Former | 1-10 |
| Glutamide based gellant | 1-10 |
| Butylene Glycol | 5-15 |
| Pigment | 5-15 |
| Preservative | 0.1-1.5 |

| **WATER PHASE** | |
|---|---|
| Water | 20-50 |
| Film Former | 1-5 |
| Chelating agent | 0.1-1 |
| Total: | 100.00 |

The mascara composition is prepared by heating the cyclomethicone, PEG-12 Dimethicone, silicone fluid, and film former to 85° C while milling on Silverson L4RT-Q Laboratory Mixer. Once solution is at 85° C the glutamide based gellant and butylene glycol are pre-heated and added. Then pigments and preservatives are slowly added until fully dispersed. The water is then pre-heated and added to batch and milled. The batch is then cooled.

### B. Comparative Mascara emulsion

A further formulation for a comparative mascara is set forth in Table 4 below.

**Table 4**

| INCI name/description | % |
|---|---|
| **OIL PHASE** | |
| Cyclomethicone | 32 |
| PEG-12 Dimethicone | 2.2 |
| Silicone Fluid | 2.2 |
| Acrylates/dimethicone copolymer | 1.6 |
| Dibutyl lauroyl glutamide | 3.3 |
| Butylene Glycol | 11 |
| Black Iron Oxide | 8.2 |
| Phenoxyethanol | 0.6 |
| **WATER PHASE** | |
| Water | 36.9 |
| Acrylates Copolymer | 1.5 |
| EDTA | 0.5 |
| Total: | 100.00 |

### C. Mascara emulsion

A formulation for a mascara of the current invention is set forth in Table 5 below.

**Table 5**

| INCI name/description | % |
|---|---|
| **OIL PHASE** | |
| Gellants | 0.3% |
| Octyldodecanol + VP hexadecene copolymer | 5.0% |
| Traditional wax (beeswax) | 5.0% |
| Silicone waxes | 5.0% |
| SE wax (beeswaxes) | 4.4% |
| Film former | 2.0% |
| Emulsifiers | 10% |
| Pigments (grind) | 0.15% |
| **WATER PHASE** | |
| Water | 43.05% |
| Film formers | 16% |
| Non-foaming agents | 0.1% |
| Thickening agent | 3.0% |
| Pigments | 0.5% |
| Effect pigments | 5.0% |
| Preservative | 0.5% |
| Total: | 100.00 |

### Example 3: Additional Exemplary Mascara Formulation

A. An anhydrous mascara composition according to the current invention is provided in Table 6.

**Table 6**

| INCI name/description | % |
|---|---|
| Octyldodecanol | Qs |
| Amino Acid Gellant(s) | 1-6 |
| Ethylenediamine/hydrogenated dimer dilinoleate alkyl amide | 1-15 |
| C9-C11 Isoparaffin | 1-10 |
| Wax Blend Combo | <25 |
| Fillers | 1-10 |
| Pigments | 1-15 |
| Film Former | 5-25 |
| Total: | 100.00 |

The mascara composition is prepared by heating the octyldodecanol to 115° C while milling on Silverson L4RT-Q Laboratory Mixer. Once octyldodecanol is at 115° C the amino acid gellant is added and milled until the solution is clear and then Ethylenediamine/Hydrogenated Dimer Dilinoleate is slowly added until fully dispersed. The remaining ingredients are then added and mixed into the resulting composition.

### B. Mascara

Another comparative mascara composition is provided below in Table 7.

**Table 7**

| **Ingredient** | **Percentage** |
|---|---|
| Phase A | |
| Octyldodecanol | 22 |
| Phase B | |
| Dibutyl Ethylhexanol Glutamide | 1.2 |
| Dibutyl Lauroyl Glutamide | 1.8 |
| Ethylenediamine/Hydrogenated Dimer Dilinoleate | 11.4 |
| Phase C | |
| C9-11 Isoparaffin | 11.6 |
| Phase D | |
| Wax Blend (Carnauba wax 32%, Kahlwax-7307 32%, Beeswax 20%, Ozokerite-170D 8%, Silwax 5022 8%) | 20 |
| Phase E | |
| Prizmalite Microspheres | 2.5 |
| Talc Italian | 2.5 |
| Phase F | |
| D&C Black No. 2/Synthetic Wax-Dispersion | 2 |
| Iron Oxide Black: | 8 |
| Ferric Blue | 0.5 |
| Phase G | |
| Acrylates Copolymer/Isododecane | 16.5 |

The mascara composition is prepared by heating the octyldodecanol to 115° C while milling on Silverson L4RT-Q Laboratory Mixer. Once octyldodecanol is at 115° C the amino acid gellant is added and milled until the solution is clear and then Ethylenediamine/Hydrogenated Dimer Dilinoleate Alkyl Amide is slowly added until fully dispersed. The remaining ingredients are then added and mixed into the resulting composition.

### Example 4: Mascaras prepared with low opacity waxes

Three different low opacity mascara emulsions were prepared according to the formulations in the tables below.

A. A comparative mascara emulsion was prepared with a low wax content of 2% as shown in Table 8.

**Table 8**

| **2% Wax Formula** | | | |
|---|---|---|---|
| Ingredients | | | Percentage |
| | **Oil Phase** | | |
| | | Gellants | 0.30% |
| | | Octyldodecanol | 2.00% |
| | | Octyldodecanol +VP hexadecene copolymer | 3.00% |
| | | Emulsifer | 6.00% |
| | | Film Formers | 2.00% |
| | | Beeswax | 0.89% |
| | | Silwax D221M | 0.36% |
| | | POE (20M) Sorbitol Beeswax | 0.10% |
| | | PEG-8 Beeswax | 0.21% |
| | | Silwax L118 | 0.43% |
| | | Fillers | 5.00% |
| | | Pigments | 0.15% |
| | | | |

| | **Water Phase** | | |
|---|---|---|---|
| | | Water | 53.56% |
| | | Film Former | 16.00% |
| | | Thickening Agents | 3.00% |
| | | Pigments | 3.50% |
| | | Emulsifier | 3.00% |
| | | preservative | 0.50% |
| Total: 100.00% | | | |

B. A mascara emulsion of the invention was prepared with a high wax content of 11% as shown in Table 9.

**Table 9**

| **11% Wax Formula** | | | |
|---|---|---|---|
| Ingredients | | | Percentage |
| | **Oil Phase** | | |
| | | Gellants | 0.30% |
| | | Octyldodecanol | 2.00% |
| | | Octyldodecanol +VP hexadecene copolymer | 3.00% |
| | | Emulsifer | 6.00% |
| | | Film Formers | 2.00% |
| | | Beeswax | 5.00% |
| | | Silwax D221M | 2.00% |
| | | POE (20M) Sorbitol Beeswax | 0.50% |
| | | PEG-8 Beeswax | 1.50% |
| | | Silwax L118 | 2.50% |
| | | Fillers | 5.00% |
| | | Pigments | 0.15% |
| | | | |

| | **Water Phase** | | |
|---|---|---|---|
| | | Water | 44.05% |
| | | Film Former | 16.00% |
| | | Thickening Agents | 3.00% |
| | | Pigments | 3.50% |
| | | Emulsifier | 3.00% |
| | | Preservative | 0.50% |
| Total: 100.00% | | | |

C. A comparative mascara emulsion was prepared with a high wax content of 15% in the absence of gellants as shown in Table 10.

**Table 10**

| **15% Wax Formula no gellants** | | | |
|---|---|---|---|
| Ingredients | | | Percentage |
| | **Oil Phase** | | |
| | | Gellants | 0.00% |
| | | Octyldodecanol | 0.00% |
| | | Octyldodecanol +VP hexadecene copolymer | 0.00% |
| | | Emulsifer | 6.00% |
| | | Film Formers | 2.00% |
| | | Beeswax | 6.71% |
| | | Silwax D221M | 2.71% |
| | | POE (20M) Sorbitol Beeswax | 0.75% |
| | | PEG-8 Beeswax | 1.58% |
| | | Silwax L118 | 3.24% |
| | | Fillers | 5.00% |
| | | Pigments | 0.15% |
| | | | |

| | **Water Phase** | | |
|---|---|---|---|
| | | Water | 45.86% |
| | | Film Former | 16.00% |
| | | Thickening Agents | 3.00% |
| | | Pigments | 3.50% |
| | | Emulsifier | 3.00% |
| | | preservative | 0.50% |
| Total: 100.00% | | | |

### Example 5: Color travel and multi-angle chroma of mascaras prepared with low opacity waxes

To test the color attributes of each of the mascara formulations described in Example 4 (2% wax + gel; 11% wax + gel; and 15% wax + no gel), samples of each were assessed for color travel and multi-angle chroma. For comparison purposes, also tested was a high-percentage opaque wax mascara that does not contain gellants.

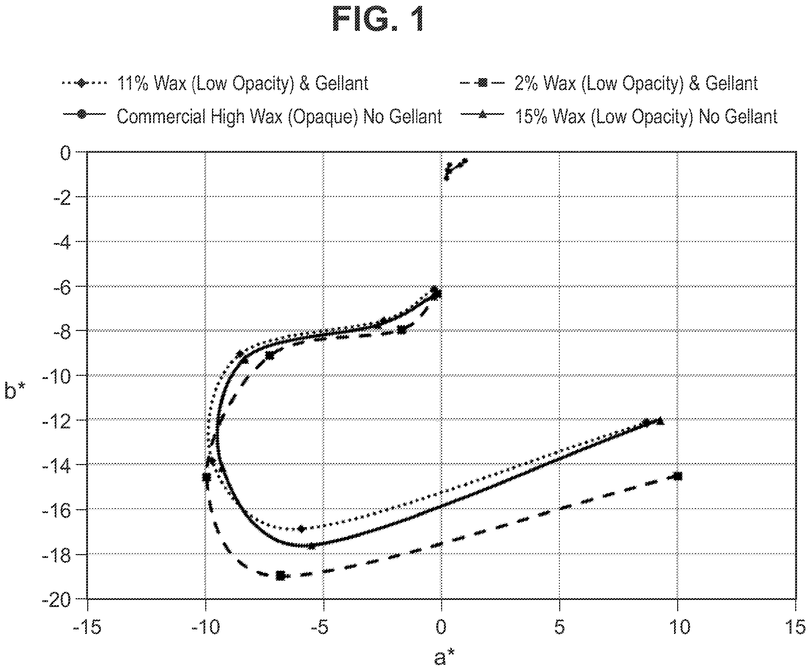

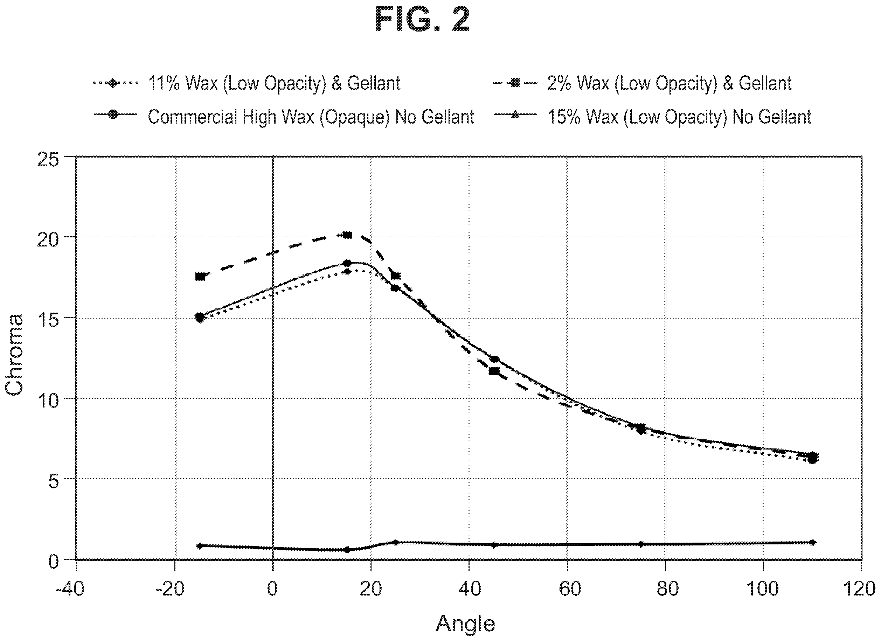

Samples of each of the three mascara formulations and the commercial formulation were drawn down as a 3 mil film onto a Leneta opacity chart, and the films were allowed to dry for 1 hour prior to measurements. Each sample was flat and free of structures. Once dried, L*, a*, b* readings and chroma readings were taken at different angles using a multi-angle spectrophotometer (BYK-mac color, Model 6340). The color travel results are presented in Figure 1 and the multi-angle chroma results are presented in Figure 2. The results show that the low opacity wax mascara formulations of the present invention exhibit better color travel and multi-angle chroma as compared to the commercial product.

### Example 6: Payoff (weight deposition) of mascaras prepared with low opacity waxes

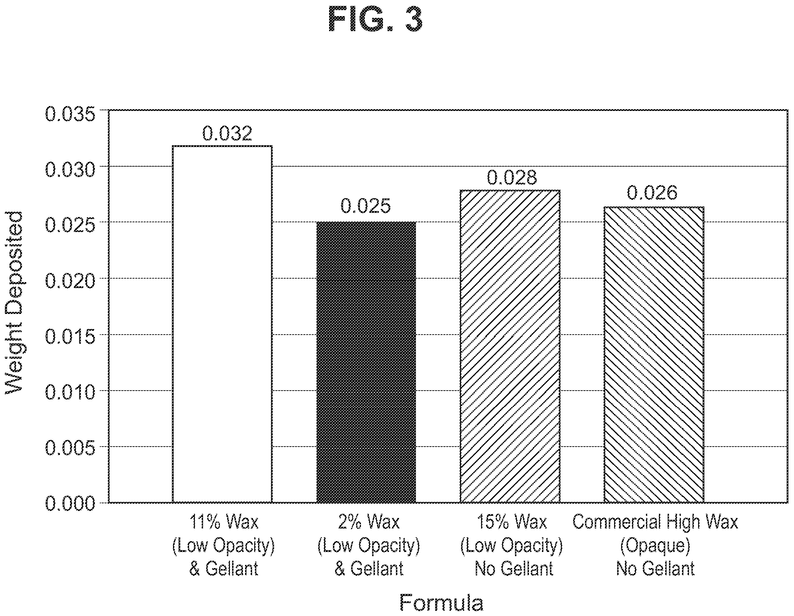

The same three mascaras described in Example 4 and the same commercial mascara described in Example 5 were assessed for payoff. To assess the payoff of a sample, a brush containing the sample was used to apply 14 strokes of the sample to a set of pre-weighed false eyelashes that were secured to a tube. The false eyelashes were allowed to dry for ten minutes, after which another 14 strokes of the sample was applied. After allowing the false eyelashes to dry for one hour, they were weighed. The weight (in grams) that was picked up after the application of the 28 total strokes was calculated as the difference between the untreated lashes and the treated lashes. The results are shown in Figure 3. The data show that the sample having high levels of low opacity wax in combination with a gellant exhibited a payoff that is greater than both the lower weight percentage wax composition and the composition that was free of gellants.

### Example 7: Payoff (weight deposition) of mascaras prepared with varying low opacity waxes

To assess how different waxes affect payoff, six mascara emulsions were prepared according to the formula below. Each formulation contained 12% wax. The six formulations contained, as the wax component, Silwax D221M; Silwax L118; Silwax Di-5026; Beeswax; Carnauba Wax; and Paraffin Wax, respectively. The formulation is presented in Table 11.

**Table 11**

| **Phase** | **Ingredient** | % **Weight** |
|---|---|---|
| **Water** | Water | 45.36 |
| **Oil** | Film formers | 20 |
| | Thickening Agents | 0.95 |
| | Emulsifier | 3.2 |
| | Preservative | 0.5 |
| | Pigments | 7.5 |
| | | |
| | Emulsifier | 5.54 |
| | Wax | 12 |
| | Thickening Agents | 4.95 |
| **Total** | | **100** |

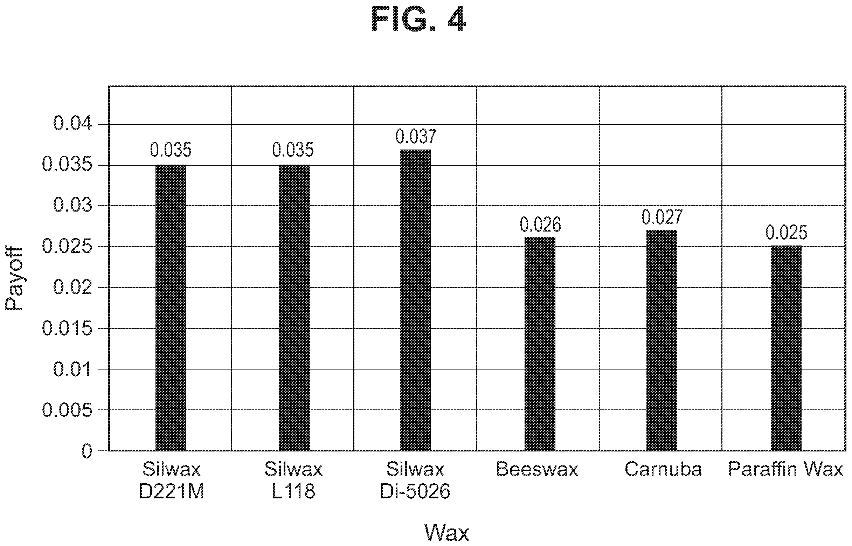

Payoff values were determined for each of the six mascara emulsions as described in Example 6. The results are presented in Figure 4. The data show that the mascara compositions prepared with silicone waxes (Silwax) exhibit superior payoff compared to mascara compositions made with other low opacity waxes.

### Example 8: Moldable mascara

Three mascara emulsions were tested to determine their moldability, as measured by a resiliency test described below. Sample A is according to the invention and Sample B and C are comparative examples. The moldability of a mascara comprising wax and gellants (Sample A) was compared to the moldability of a mascara that lacked waxes (Sample B) and a mascara that lacked gellants (Sample C). The formulations for each mascara are provided below in Table 12.

**Table 12**

| Ingredient | A | B | C |
|---|---|---|---|
| | Weight % | | |
| Aqueous Phase | | | |
| Water | *q.s.* | *q.s.* | *q.s.* |
| Triethanolamine | 2.00 | 2.00 | 2.00 |
| Sequestering agent | 0.25 | 0.25 | 0.25 |
| Thickeners | 3.10 | 3.10 | 3.10 |
| Iron Oxides | 0.50 | 0.50 | 0.50 |
| Film former | 16.0 | 16.0 | 16.0 |
| Preservative | 0.50 | 0.50 | 0.50 |

| Oil Phase | | | |
|---|---|---|---|
| Octyldodecanol | 1.67 | 1.67 | 1.67 |
| Octyldodecanol and VP/hexadecene copolymer | 3.33 | 3.33 | 3.33 |
| Dibutyl Ethylhexanoyl Glutamide | 0.15 | 0.15 | -- |
| Dibutyl Lauroyl Glutamide | 0.15 | 0.15 | -- |
| Silicone wax | 4.4 | -- | 4.4 |
| Beeswax | 4.00 | -- | 4.00 |
| POE (20M) Sorbitol Beeswax | 0.60 | -- | 0.60 |
| PEG-8 Beeswax | 1.20 | -- | 1.20 |
| Film former | 2.00 | 2.00 | 2.00 |
| Emulsifier | 4.00 | 4.00 | 4.00 |
| Shine agent | 1.5 | 1.5 | 1.5 |
| Stearic acid | 4.00 | 4.00 | 4.00 |
| D&C Black #2* (carbon black) | 2.00 | 2.00 | 2.00 |
| Rheology modifier | 1.00 | 1.00 | 1.00 |
| Particulate fillers | 3 | 3 | 3 |
| total | 100 | 100 | 100 |

| | | | |
|---|---|---|---|
| *As pigment grind (1:5 w/w in oil) | | | |

### Resiliency Test

A resiliency test was used to assess the moldability of dry films of each of the three mascaras. Films of the mascara emulsions were prepared to be 1 mm thick, and were allowed to dry at 37°C for 24 hours. The dry films were placed onto a film support rig that was fitted to a heavy duty platform of a TA-XT2 Texture Analyzer (Stable Micro Systems). The Texture Analyzer was equipped with a 1/4 inch probe that was used to apply a downward target force of 2.0 g to the dry films, at a speed of 0.20 mm/sec, at a displacement distance of 2.0 mm. The force was then withdrawn from the films at 0.2 mm/sec, allowing the films to recover and exert an upward force on the probe. The compression in g·mm and the recovery in g·mm were recorded. Resiliency was calculated as the percentage of the compression that recovered. Resiliency is a measure of how moldable the films are. The results are presented below in Table 13.

**Table 13**

| **Sample** | **Force at Target Distance (g)** | **Compression (g·mm)** | **Recovery (g·mm)** | **Resilience (%)** |
|---|---|---|---|---|
| A | 7.52 | 38.48 | 2.58 | 6.69 |
| B | 26.45 | 125.26 | 15.04 | 12.01 |
| C | 19.10 | 104.00 | 10.40 | 10.00 |

The dry films of the mascara formulation of Sample A required the least amount of force to compress, and once compressed, showed the least recovery, and the least resilience, indicating that it is the most moldable mascara compared to the otherwise identical mascaras without wax (Sample B), or without gellants (Sample C). In addition, the mascara without gellants (Sample C) was less resilient and more moldable than the mascara without waxes (Sample B).

The effect of pigment load on moldability was also assessed. At a constant particulate filler content of 3%, samples D, E, and F varied the amount of iron oxide pigment by 0.5%, 3.0%, and 7.0%, respectively, for a total particulate content ranging from 3.5% - 10%. The formulations are below in Table 14.

**Table 14**

| Ingredient | D | E | F |
|---|---|---|---|
| | Weight % | | |
| Aqueous Phase | | | |
| Water | *q.s.* | *q.s.* | *q.s.* |
| Triethanolamine | 2.00 | 2.00 | 2.00 |
| Sequestering agent | 0.25 | 0.25 | 0.25 |
| Thickeners | 3.10 | 3.10 | 3.10 |
| Iron Oxides | 0.50 | 3.0 | 7.0 |
| Film former | 16.0 | 16.0 | 16.0 |
| Preservative | 0.50 | 0.50 | 0.50 |

| Oil Phase | | | |
|---|---|---|---|
| Octyldodecanol | 1.67 | 1.67 | 1.67 |
| Octyldodecanol and VP/hexadecene copolymer | 3.33 | 3.33 | 3.33 |
| Dibutyl Ethylhexanoyl Glutamide | 0.15 | 0.15 | 0.15 |
| Dibutyl Lauroyl Glutamide | 0.15 | 0.15 | 0.15 |
| Silicone wax | 4.4 | 4.4 | 4.4 |
| Beeswax | 4.00 | 4.00 | 4.00 |
| POE (20M) Sorbitol Beeswax | 0.60 | 0.60 | 0.60 |
| PEG-8 Beeswax | 1.20 | 1.20 | 1.20 |
| Film former | 2.00 | 2.00 | 2.00 |
| Emulsifier | 4.00 | 4.00 | 4.00 |
| Shine agent | 1.5 | 1.5 | 1.5 |
| Stearic acid | 4.00 | 4.00 | 4.00 |
| Rheology modifier | 1.00 | 1.00 | 1.00 |
| Particulate fillers | 3 | 3 | 3 |
| total | 100 | 100 | 100 |

As shown by the data below in Table 15 below, the resilience percentage behaves non-linearly, at total particulate levels between about 6% and about 10% by weight. Accordingly, in some embodiments, the amount of total particulate in the composition will be less than 10%, or less than 9%, or less than 8%, or less than 7%, or less than 6% by weight.

**Table 15**

| **Sample** | **Force at Target Distance (g)** | **Compression (g·mm)** | **Recovery (g·mm)** | **Resilience (%)** |
|---|---|---|---|---|
| Sample D (0.5% iron oxide) | 7.90 | 49.47 | 4.12 | 8.33 |
| Sample E (3.0% iron oxide) | 8.53 | 51.73 | 4.55 | 8.80 |
| Sample F (7.0% iron oxide) | 9.49 | 47.77 | 5.80 | 12.14 |

## Claims

1. A method for styling keratin fibers comprising:
applying to keratin fibers a composition capable of forming a film, which upon evaporation of solvents is **characterized by** a resilience of less than 15% when displaced 2.0 mm by a compression force at 25°C;
allowing said composition to dry on said keratin fibers to form a film on at least a portion of the surface of said fibers; and
molding said keratin fibers into a desired configuration by applying a force thereto, wherein said keratin fibers remain in said desired configuration after said force is removed
wherein said composition comprises
(a) from 7.5 to 30% by weight of a wax component comprising one or more waxes selected from the group consisting of carnauba wax, beeswax, ozokerite and silicone wax,
(b) between 0.1% and 1% of an oil phase gellant comprising dibutyl laurolyl glutamide and/or dibutyl ethylhexanoyl glutamide,
(c) less than 15% by weight of one or more pigments and/or fillers, and
(d) between 1% to 10% of an oil capable of forming a gel with the oil phase gellant comprising a compound selected from the group consisting of vegetable oils, octyl palmitate, isopropyl myristate, isopropyl palmitate, dicapryl ether, fatty alcohols, isoparaffins, silicone oils, and hydrocarbon oils.

2. The method according to claim 1, wherein subsequent to said step of molding, remolding said keratin fibers into a second desired configuration by applying a second force thereto, wherein said keratin fibers remain in said second configuration after said second force is removed.

3. The method according to claim 1 or 2, wherein said step of molding occurs in the absence of heat.

4. The method according to any one of claims 1 to 3, wherein said composition comprises:
(a) from 7.5 to 15% by weight of the wax component comprising one or more waxes selected from the group consisting of carnauba wax, beeswax, ozokerite and silicone wax;
(b) between 0.1% and 1% of the oil phase gellant comprising dibutyl laurolyl glutamide and/or dibutyl ethylhexanoyl glutamide;
(c) less than 15% by weight of the one or more pigments and/or fillers; and
(d) between 1% to 10% of the oil capable of forming a gel with the oil phase gellant comprising a compound selected from the group consisting of vegetable oils, octyl palmitate, isopropyl myristate, isopropyl palmitate, dicapryl ether, fatty alcohols, isoparaffins, silicone oils, and hydrocarbon oils.

5. The method according to any one of claims 1 to 4, wherein said resilience is less than 12% or wherein said resilience is less than 8%.

6. The method according to any one of claims 1 to 5, wherein the pigments and/or fillers in said composition comprise less than 10% by weight, or less than 7% by weight, or less than 4% by weight.

7. The method according to any one of claims 1 to 6, wherein the composition applied is a mascara.

8. The method according to any one of claims 1 to 7, wherein the keratin fibers are eye lashes.

9. The method according to any one of claims 1 to 8, wherein the pigments and/or fillers in said composition comprises iron oxide and/or carbon black.

10. The method according to any one of claims 1 to 9, wherein the wax component in said composition comprises silicone wax.

11. The method according to any one of claims 1 to 10, wherein the composition further comprises at least one film-forming agent.

12. A cosmetic composition for styling keratin fibers, the composition comprising
(a) from 7.5 to 30% by weight of a wax component comprising one or more waxes selected from the group consisting of carnauba wax, beeswax, ozokerite and silicone wax,
(b) between 0.1% and 1% of an oil phase gellant comprising dibutyl laurolyl glutamide and/or dibutyl ethylhexanoyl glutamide,
(c) less than 15% by weight of one or more pigments and/or fillers, and
(d) between 1% to 10% of an oil capable of forming a gel with the oil phase gellant comprising a compound selected from the group consisting of vegetable oils, octyl palmitate, isopropyl myristate, isopropyl palmitate, dicapryl ether, fatty alcohols, isoparaffins, silicone oils, and hydrocarbon oils.

13. The cosmetic composition as claimed in claim 12, wherein the composition comprises:
(a) from 7.5 to 15% by weight of the wax component comprising one or more waxes selected from the group consisting of carnauba wax, beeswax, ozokerite and silicone wax;
(b) between 0.1% and 1% of the oil phase gellant comprising dibutyl laurolyl glutamide and/or dibutyl ethylhexanoyl glutamide;
(c) less than 15% by weight of one or more pigments and/or fillers; and
(d) between 1% to 10% of an oil capable of forming a gel with the oil phase gellant comprising a compound selected from the group consisting of vegetable oils, octyl palmitate, isopropyl myristate, isopropyl palmitate, dicapryl ether, fatty alcohols, isoparaffins, silicone oils, and hydrocarbon oils.

14. The cosmetic composition as claimed in claims 12 and 13, wherein the composition is further defined by claims 6, 7, 9, 10 and 11.

15. The method according to claims 1-11 and the cosmetic composition according to claims 12-14, wherein the wax component has a low opacity, the waxes of the wax component collectively having a ΔL* value of less than 8 when determined as follows:
a cosmetic gelbase of Dibutyl Ethylhexanoyl Glutamide 1.19%, Dibutyl Lauroyl Glutamide 1.785%, Ethylenediamine/Hydrogenated Dimer Dilinoleate Alkyl Amide 19.05%; Octyldodecanol 77.975% was prepared for use as a negative control and for determining the suitability of various waxes;
10% of the octyldodecanol in the gelbase is replaced by wax and the sample drawn down (3 mL) on black Leneta cards and allowed to dry for 2 hours;
once dried, five L* readings were calculated per sample using a Konica Minolta CM-2600d spectrophotometer and the data averages were calculated and a ΔL relative to the control is determined.

## Patentansprüche

1. Verfahren zum Styling von Keratinfasern enthaltend:
auf die Keratinfasern Aufbringen einer Komposition, die einen Film formen kann, die nach Verdunstung der Lösemittel durch eine Widerstandsfähigkeit von weniger als 15%, wenn sie durch eine Kompressionskraft bei 25 °C um 2,0 mm verformt wird, charakterisiert ist;
Erlauben der Trocknung der Komposition auf den Keratinfasern, so dass ein Film auf mindestens einem Teil der Oberfläche der Keratinfasern entsteht; und
Formen der Keratinfasern in eine gewünschte Konfiguration durch das Ausüben einer Kraft auf die Keratinfasern, wobei die Keratinfasern in der gewünschten Konfiguration verbleiben nachdem die Kraft entfernt ist,
wobei die Komposition enthält
(a) von 7,5 bis 30 Gewichtsprozent einer Wachskomponente, die ein oder mehrere Wachse ausgewählt aus der Gruppe bestehend aus Carnaubawachs, Bienenwachs, Ozokerit und Silikonwachs enthält,
(b) zwischen 0,1% und 1% eines Ölphasengelierungsmittels, das Dibutyllaurolylglutamid und/oder Dibutylethylhexanoylglutamid enthält,
(c) weniger als 15 Gewichtsprozent eines oder mehrerer Pigmente und/oder Füllmittel, und
(d) zwischen 1% bis 10% eines Öls, das ein Gel mit dem Ölphasengelierungsmittel bilden kann, das eine Verbindung ausgewählt aus der Gruppe bestehend aus Gemüseölen, Hexadekanonsäure-2-ethylhexylester, Myristinsäureisopropylester, Propan-2-ylhexadekanonat, Dikaprylether, Fettalkoholen, Isoparaffinen, Silikonölen, und Kohlenwasserstoffölen enthält.

2. Verfahren nach Anspruch 1, wobei nach dem Schritt des Formens die Keratinfasern erneut in eine zweite gewünschte Konfiguration durch das Ausüben einer zweiten Kraft auf die Keratinfasern geformt werden, wobei die Keratinfasern in der zweiten Konfiguration verbleiben nachdem die zweite Kraft entfernt ist.

3. Verfahren nach einem der Ansprüche 1 oder 2, wobei der Schritt des Formens in Abwesenheit von Hitze erfolgt.

4. Verfahren nach einem der Ansprüche 1 bis 3, wobei die Komposition enthält:
(a) von 7,5 bis 15 Gewichtsprozent der Wachskomponente, die ein oder mehrere Wachse ausgewählt aus der Gruppe bestehend aus Carnaubawachs, Bienenwachs, Ozokerit und Silikonwachs enthält;
(b) zwischen 0,1% und 1% des Ölphasengelierungsmittels, das Dibutyllaurolylglutamid und/oder Dibutylethylhexanoylglutamid enthält;
(c) weniger als 15 Gewichtsprozent des einen oder der mehreren Pigmente und/oder Füllmittel; und
(d) zwischen 1% bis 10% des Öls, das ein Gel mit dem Ölphasengelierungsmittel bilden kann, das eine Verbindung ausgewählt aus der Gruppe bestehend aus Gemüseölen, Hexadekanonsäure-2-ethylhexylester, Myristinsäureisopropylester, Propan-2-ylhexadekanonat, Dikaprylether, Fettalkoholen, Isoparaffinen, Silikonölen, und Kohlenwasserstoffölen enthält.

5. Verfahren nach einem der Ansprüche 1 bis 4, wobei die Widerstandsfähigkeit weniger als 12% ist oder wobei die Widerstandsfähigkeit weniger als 8% ist.

6. Verfahren nach einem der Ansprüche 1 bis 5, wobei die Pigmente und/oder Füllmittel in der Komposition weniger als 10 Gewichtsprozent oder weniger als 7 Gewichtsprozent oder weniger als 4 Gewichtsprozent ausmachen.

7. Verfahren nach einem der Ansprüche 1 bis 6, wobei die aufgebrachte Komposition eine Wimperntusche ist.

8. Verfahren nach einem der Ansprüche 1 bis 7, wobei die Keratinfasern Wimpern sind.

9. Verfahren nach einem der Ansprüche 1 bis 8, wobei die Pigmente und/oder Füllmittel in der Komposition Eisenoxid und/oder Ruß enthalten.

10. Verfahren nach einem der Ansprüche 1 bis 9, wobei die Wachskomponente in der Komposition Silikonwachs enthält.

11. Verfahren nach einem der Ansprüche 1 bis 10, wobei die Komposition weiter mindestens ein Filmformungsmittel enthält.

12. Kosmetische Komposition zum Styling von Keratinfasern, wobei die Komposition enthält
(a) von 7,5 bis 30 Gewichtsprozent einer Wachskomponente, die ein oder mehrere Wachse ausgewählt aus der Gruppe bestehend aus Carnaubawachs, Bienenwachs, Ozokerit und Silikonwachs enthält,
(b) zwischen 0,1% und 1% eines Ölphasengelierungsmittels, das Dibutyllaurolylglutamid und/oder Dibutylethylhexanoylglutamid enthält,
(c) weniger als 15 Gewichtsprozent eines oder mehrerer Pigmente und/oder Füllmittel, und
(d) zwischen 1% bis 10% eines Öls, das ein Gel mit dem Ölphasengelierungsmittel bilden kann, das eine Verbindung ausgewählt aus der Gruppe bestehend aus Gemüseölen, Hexadekanonsäure-2-ethylhexylester, Myristinsäureisopropylester, Propan-2-ylhexade-kanonat, Dikaprylether, Fettalkoholen, Isoparaffinen, Silikonölen, und Kohlenwasserstoffölen enthält.

13. Kosmetische Komposition nach Anspruch 12, wobei die Komposition enthält
(a) von 7,5 bis 15 Gewichtsprozent der Wachskomponente, die ein oder mehrere Wachse ausgewählt aus der Gruppe bestehend aus Carnaubawachs, Bienenwachs, Ozokerit und Silikonwachs enthält,
(b) zwischen 0,1% und 1% des Ölphasengelierungsmittels, das Dibutyllaurolylglutamid und/oder Dibutylethylhexanoylglutamid enthält,
(c) weniger als 15 Gewichtsprozent des einen oder der mehreren Pigmente und/oder Füllmittel, und
(d) zwischen 1% bis 10% des Öls, das ein Gel mit dem Ölphasengelierungsmittel bilden kann, das eine Verbindung ausgewählt aus der Gruppe bestehend aus Gemüseölen, He-xadekanonsäure-2-ethylhexylester, Myristinsäureisopropylester, Propan-2-ylhexadeka-nonat, Dikaprylether, Fettalkoholen, Isoparaffinen, Silikonölen, und Kohlenwasserstoffölen enthält.

14. Kosmetische Komposition nach Ansprüchen 12 und 13, wobei die Komposition weiter durch Ansprüche 6, 7, 9, 10 und 11 definiert ist.

15. Verfahren nach einem der Ansprüche 1 bis 11 und die kosmetische Komposition nach einem der Ansprüche 12 bis 14, wobei die Wachskomponente eine geringe Durchsichtigkeit hat, wobei die Wachse der Wachskomponente zusammen einen ΔL^{∗} Wert von weniger als 8 hat, wenn er wie folgt bestimmt wird:
eine kosmetische Gelbase aus Dibutylethylhexanoylglutamid 1,19%, Dibutyllaurolylglutamid 1,785%, Ethylendiamin/hydriertes dimeres Dilinoleatalkylamid 19,05%, Octyldodecanol 77,975% wurde zur Verwendung als negatives Kontrollexperiment und zur Feststellung der Eignung verschiedener Wachse hergestellt;
10% des Octyldodecanol in der Gelbase wird mit Wachs ersetzt, die Probe (3 mL) auf schwarzen Lenetakarten abgezogen und für 2 Stunden getrocknet wird;
wenn die Probe getrocknet ist, wurden 5 L^{∗} Lesungen pro Probe mit einem Konica Minolta CM-2600d Fotospektrometer und die Datenmittel berechnet und ein ΔL relativ zur Kontrolle bestimmt.

## Revendications

1. Procédé pour le coiffage de fibres de kératine comprenant :
l'application à des fibres de kératine d'une composition apte à former un film, qui, lors de l'évaporation de solvants, est **caractérisé par** une résilience inférieure à 15 % lorsqu'il est déplacé de 2,0 mm par une force de compression à 25°C ;
laisser ladite composition sécher sur lesdites fibres de kératine pour former un film sur au moins une partie de la surface desdites fibres ; et
mouler lesdites fibres de kératine selon une configuration souhaitée par l'application d'une force sur celles-ci, dans lequel lesdites fibres de kératine restent dans ladite configuration souhaitée après que ladite force est retirée
dans lequel ladite composition comprend
(a) de 7,5 à 30 % en poids d'un composant de cire comprenant une ou plusieurs cires choisies dans le groupe consistant en la cire de carnauba, la cire d'abeille, l'ozocérite et la cire de silicone,
(b) entre 0,1 % et 1 % d'un gélifiant de phase huileuse comprenant du dibutyle laurolyl glutamide et/ou du dibutyle éthylhexanoyl glutamide,
(c) moins de 15 % en poids d'un ou plusieurs pigments et/ou charges, et
(d) entre 1 % et 10 % d'une huile apte à former un gel avec le gélifiant de phase huileuse comprenant un composé choisi dans le groupe consistant en les huiles végétales, le palmitate d'octyle, le myristate d'isopropyle, le palmitate d'isopropyle, l'éther dicaprylique, les alcools gras, les isoparaffines, les huiles de silicone, et les huiles hydrocarbonées.

2. Procédé selon la revendication 1, comprenant en outre, à la suite de ladite étape de moulage, une étape de remoulage desdites fibres de kératine selon une deuxième configuration souhaitée par l'application d'une deuxième force sur celles-ci, dans lequel lesdites fibres de kératine restent dans ladite deuxième configuration après que ladite deuxième force est retirée.

3. Procédé selon la revendication 1 ou 2, dans lequel ladite étape de moulage se produit en l'absence de chaleur.

4. Procédé selon l'une quelconque des revendications 1 à 3, dans lequel ladite composition comprend :
(a) de 7,5 à 15 % en poids du composant de cire comprenant une ou plusieurs cires choisies dans le groupe consistant en la cire de carnauba, la cire d'abeille, l'ozocérite et la cire de silicone ;
(b) entre 0,1 % et 1 % du gélifiant de phase huileuse comprenant du dibutyle du laurolyl glutamide et/ou du dibutyle éthylhexanoyl glutamide ;
(c) moins de 15 % en poids du ou des pigments et/ou charges ; et
(d) entre 1 % et 10 % de l'huile apte à former un gel avec le gélifiant de phase huileuse comprenant un composé choisi dans le groupe consistant en les huiles végétales, le palmitate d'octyle, le myristate d'isopropyle, le palmitate d'isopropyle, l'éther dicaprylique, les alcools gras, les isoparaffines, les huiles de silicone, et les huiles hydrocarbonées.

5. Procédé selon l'une quelconque des revendications 1 à 4, dans lequel ladite résilience est inférieure à 12 % ou dans lequel ladite résilience est inférieure à 8 %.

6. Procédé selon l'une quelconque des revendications 1 à 5, dans lequel les pigments et/ou charges dans ladite composition comprennent moins de 10 % en poids, ou moins de 7 % en poids, ou moins de 4 % en poids.

7. Procédé selon l'une quelconque des revendications 1 à 6, dans lequel la composition appliquée est un mascara.

8. Procédé selon l'une quelconque des revendications 1 à 7, dans lequel les fibres de kératine sont des cils.

9. Procédé selon l'une quelconque des revendications 1 à 8, dans lequel les pigments et/ou charges dans ladite composition comprennent de l'oxyde de fer et/ou du noir de carbone.

10. Procédé selon l'une quelconque des revendications 1 à 9, dans lequel le composant de cire dans ladite composition comprend de la cire de silicone.

11. Procédé selon l'une quelconque des revendications 1 à 10, dans lequel la composition comprend en outre au moins un agent filmogène.

12. Composition cosmétique pour le coiffage de fibres de kératine, la composition comprenant
(a) de 7,5 à 30 % en poids d'un composant de cire comprenant une ou plusieurs cires choisies dans le groupe consistant en la cire de carnauba, la cire d'abeille, l'ozocérite et la cire de silicone,
(b) entre 0,1 % et 1 % d'un gélifiant de phase huileuse comprenant du dibutyle laurolyl glutamide et/ou du dibutyle éthylhexanoyl glutamide,
(c) moins de 15 % en poids d'un ou plusieurs pigments et/ou charges, et
(d) entre 1 % et 10 % d'une huile apte à former un gel avec le gélifiant en phase huileuse comprenant un composé choisi dans le groupe consistant en les huiles végétales, le palmitate d'octyle, le myristate d'isopropyle, le palmitate d'isopropyle, l'éther dicaprylique, les alcools gras, les isoparaffines, les huiles de silicone, et les huiles hydrocarbonées.

13. Composition cosmétique selon la revendication 12, dans laquelle la composition comprend :
(a) de 7,5 à 15 % en poids du composant de cire comprenant une ou plusieurs cires choisies dans le groupe consistant en la cire de carnauba, la cire d'abeille, l'ozocérite et la cire de silicone ;
(b) entre 0,1 % et 1 % du gélifiant de phase huileuse comprenant du dibutyle laurolyl glutamide et/ou du dibutyle éthylhexanoyl glutamide ;
(c) moins de 15 % en poids d'un ou plusieurs pigments et/ou charges ; et
(d) entre 1 % et 10 % d'une huile apte à former un gel avec le gélifiant de phase huileuse comprenant un composé choisi dans le groupe consistant en les huiles végétales, le palmitate d'octyle, le myristate d'isopropyle, le palmitate d'isopropyle, l'éther dicaprylique, les alcools gras, les isoparaffines, les huiles de silicone, et les huiles hydrocarbonées.

14. Composition cosmétique selon l'une quelconque des revendications 12 et 13, dans laquelle la composition est définie en outre par les revendications 6, 7, 9, 10 et 11.

15. Procédé selon les revendications 1-11 et composition cosmétique selon les revendications 12-14, dans lequel le composant de cire présente une faible opacité, les cires du composant de cire présentant collectivement une valeur ΔL* inférieure à 8 lorsqu'elle est déterminée de la manière suivante :
une base de gel cosmétique de dibutyle éthylhexanoyl glutamide à 1,19 %,de dibutyle lauroyl glutamide à 1,785 %, d'éthylènediamine/alkyle amide de dilinoléate dimère hydrogéné à 19,05 %, octyldodécanol à 77,975 % a été préparée pour une utilisation en tant que contrôle négatif et pour la détermination de l'adéquation de diverses cires ;
10 % de l'octyldodécanol dans la base de gel sont remplacés par de la cire et l'échantillon déposé (3 ml) sur des cartes Leneta noires et laissé sécher pendant 2 heures ;
une fois séché, cinq lectures L* ont été calculées par échantillon au moyen d'un spectrophotomètre Konica Minolta CM-2600d et les moyennes de données ont été calculées et un ΔL par rapport au contrôle est déterminé.
